(19) Europäisches Patentamt
European Patent Office
Office européen des brevets

(11) Veröffentlichungsnummer: **0 217 742**
**A2**

# (12) EUROPÄISCHE PATENTANMELDUNG

(21) Anmeldenummer: 86810364.9

(22) Anmeldetag: 15.08.86

(51) Int. Cl.⁴: **C 07 D 493/22**
A 01 N 43/22, A 23 K 1/17
//(C07D493/22, 315:00, 311:00, 311:00, 307:00)

(30) Priorität: 23.08.85 CH 3642/85

(43) Veröffentlichungstag der Anmeldung: 08.04.87 Patentblatt 87/15

(84) Benannte Vertragsstaaten: AT BE CH DE FR GB IT LI LU NL SE

(71) Anmelder: **CIBA-GEIGY AG**
**Klybeckstrasse 141**
**CH-4002 Basel(CH)**

(72) Erfinder: **Maienfisch, Peter, Dr.**
**Traugott Meyer-Strasse 5**
**CH-4147 Aesch(CH)**

(72) Erfinder: **Sturm, Elmar, Dr.**
**Klusstrasse 66**
**CH-4147 Aesch(CH)**

## (54) 5-Ester von Milbemycinen zur Bekämpfung von parasitären Schädlingen.

(57) Es werden ekto- und endoparasitizid wirkende Milbemycine der Formel I

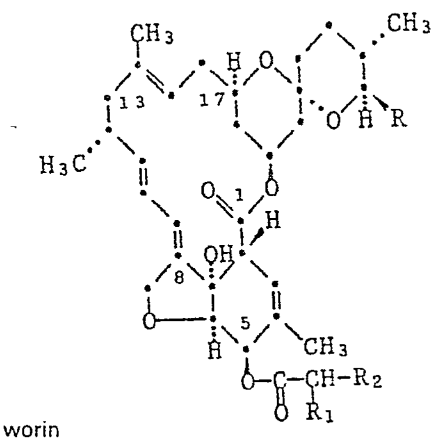

(I)

worin
R für Methyl, Ethyl Isopropyl oder sek.-Butyl steht;
$R_1$ für Wasserstoff, Fluor oder $C_1$-$C_4$-Alkyl steht;
$R_2$ eine der Gruppen

$$\left(-X-\underset{\underset{O}{\|}}{C}-CH_2\right)_n R_3 \text{ oder } -X-\underset{\underset{O}{\|}}{C}-R_4$$

repräsentiert; wobei
n eine der Zahlen 2 bis 6 bedeutet;
X für Sauerstoff oder Schwefel steht ;
$R_3$ für Halogen steht; und
$R_4$ Wasserstoff, $C_1$-$C_{12}$-Alkoxy, $C_3$-$C_7$-Cycloalkoxy, $C_2$-$C_6$-Alkenyloxy, unsubstituiertes oder durch Halogen, $C_1$-$C_4$-Alkoxy, Hydroxy und/oder COOG substituiertes $C_1$-$C_{18}$-Alkyl, wobei G für Wasserstoff, ein Alkalimetall- oder Erdalkalimetallkation steht, unsubstituiertes oder durch Halogen und oder $C_1$-$C_4$-Alkyl substituiertes $C_3$-$C_7$-Cycloalkyl, unsubstituiertes oder durch Halogen substituiertes $C_2$-$C_{18}$-Alkenyl, unsubstituiertes oder durch Halogen substituiertes $C_2$-$C_{18}$-Alkinyl, unsubstituiertes oder durch Halogen, $C_1$-$C_4$-Alkyl, $C_1$-$C_4$-Alkoxy, Nitro, Cyano und/oder $C_1$-$C_4$-Haloalkyl substituiertes Phenyl oder einen unsubstituierten oder durch Oxo und/oder ein- bis dreifach durch Halogen, $C_1$-$C_4$-Alkyl, $C_1$-$C_4$-Alkoxy und/oder $C_1$-$C_4$-Haloalkyl substituierten fünf- oder sechsgliedrigten, ungesättigen oder gesättigten heterocyclischen Ring mit ein- bis drei Heteroatomen ausgewählt aus der Reihe Stickstoff, Sauerstoff und Schwefel bedeutet, beschrieben, sowie deren Anwendung im Agrarbereich und ihre Herstellung durch Veresterung der zugrundeliegenden 5-OH-Milbemycine.

EP 0 217 742 A2

Croydon Printing Company Ltd.

0217742

CIBA-GEIGY AG
Basel (Schweiz)

5-15475/=

# 5-Ester von Milbemycinen zur Bekämpfung von parasitären Schädlingen

Die vorliegende Erfindung betrifft in 5-Position veresterte Milbemycin-Derivate der nachstehenden Formel I, ihre Herstellung und ihre Verwendung zur Bekämpfung von Schädlingen wie Ekto- und Endoparasiten, insbesondere gegen Nematoden, ferner Schädlingsbekämpfungsmittel, die mindestens eine dieser Verbindungen als Wirkstoff enthalten.

Die erfindungsgemässen Verbindungen haben die Formel I

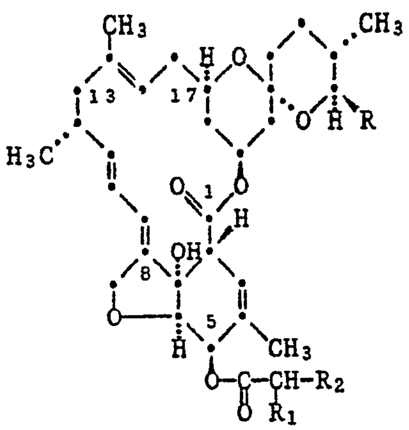

(I)

worin

R für Methyl, Ethyl, Isopropyl oder sek.-Butyl steht;

$R_1$ für Wasserstoff, Fluor oder $C_1$-$C_4$-Alkyl steht;

$R_2$ eine der Gruppen

$$-\left[X-\underset{\overset{\|}{O}}{C}-CH_2\right]_n-R_3 \quad \text{oder} \quad -X-\underset{\overset{\|}{O}}{C}-R_4$$ repräsentiert; wobei

n eine der Zahlen 2 bis 6 bedeutet;

X für Sauerstoff oder Schwefel steht ;

$R_3$ für Halogen steht; und

$R_4$ Wasserstoff, $C_1$-$C_{12}$-Alkoxy, $C_3$-$C_7$-Cycloalkoxy, $C_2$-$C_6$-Alkenyloxy, unsubstituiertes oder durch Halogen, $C_1$-$C_4$-Alkoxy, Hydroxy und/oder COOG substituiertes $C_1$-$C_{18}$-Alkyl, wobei G für Wasserstoff, ein Alkalimetall- oder Erdalkalimetallkation steht, unsubstituiertes oder durch Halogen und/oder $C_1$-$C_4$-Alkyl substituiertes $C_3$-$C_7$-Cycloalkyl, unsubstituiertes oder durch Halogen substituiertes $C_2$-$C_{18}$-Alkenyl, unsubstituiertes oder durch Halogen substituiertes $C_2$-$C_{18}$-Alkinyl, unsubstituiertes oder durch Halogen, $C_1$-$C_4$-Alkyl, $C_1$-$C_4$-Alkoxy, Nitro, Cyano und/oder $C_1$-$C_4$-Haloalkyl substituiertes Phenyl oder einen unsubstituierten oder durch Oxo und/oder ein- bis dreifach durch Halogen, $C_1$-$C_4$-Alkyl, $C_1$-$C_4$-Alkoxy und/oder $C_1$-$C_4$-Haloalkyl substituierten fünf- oder sechsgliedrigen, ungesättigten oder gesättigten heterocyclischen Ring mit ein- bis drei Heteroatomen ausgewählt aus der Reihe Stickstoff, Sauerstoff und Schwefel bedeutet.

Unter dem Begriff Alkyl selbst oder als Bestandteil eines anderen Substituenten sind je nach Zahl der angegebenen Kohlenstoffatome beispielsweise folgende geradkettige Gruppen zu verstehen: Methyl, Ethyl, Propyl, Butyl, Pentyl, Hexyl, Heptyl, Octyl, Nonyl, Decyl, Undecyl, Dodecyl, Tridecyl, Tetradecyl, Pentadecyl, Hexadecyl, Heptadecyl, Octadecyl usw. sowie ihre verzweigten Isomeren, wie z.B. Isopropyl, Isobutyl, tert.-Butyl, Isopentyl usw.. Alkenyl steht selbst oder als Bestandteil eines Alkenyloxy z.B. für Propenyl-(1), Allyl, Butenyl-(1), Butenyl-(2), Butenyl-(3) usw. Alkinyl steht beispielsweise für Ethinyl, Propinyl-(1), Propargyl, Butinyl-(1), usw. Unter Halogen soll hier und im folgenden Fluor, Chlor, Brom oder Jod verstanden werden, vorzugsweise Chlor oder Brom. Beispiele für Cycloalkyl selbst oder als Bestandteil von Cycloalkoxy sind Cyclopropyl, Cyclobutyl, Cyclopentyl, Cyclohexyl oder Cycloheptyl. Haloalkyl repräsentiert einen ein- bis perhalogenierten Alkylrest, wie z.B. $CH_2J$, $CH_2Br$, $CH_2Cl$, $CH_2F$, $CHCl_2$, $CHF_2$, $CCl_3$, $CBr_3$, $CF_3$, $C_2F_5$, $C_2Cl_5$, CFClBr, usw., wobei der Rest auch gleichzeitig durch unterschiedliche Halogenatome substituiert sein kann; bevorzugt ist

jedoch der $CF_3$-Rest. Typische fünfgliedrige heterocyclische Ringe sind:.Furan, Thiophen, Pyrrol, Isoxazol, Isothiazol, Furazan, Imidazol, 1,2,4-Triazol, 1,2,3-Triazol, Pyrazol, Pyrrolin, Oxazol, Thiazol, Thiadiazole, Pyrazolin, Thiazolin, Pyrazolidin, Pyrrolidin, Oxazolidin, Thiazolidin, Oxadiazol, Imidazolin, Imidazolidin, Pyrazolidin, Tetrahydrofuran; und typische sechsgliedgrige heterocyclische Ringe sind Pyridin, Pyridazin, Pyrimidin, Pyrazin, Thiazin, Thiadiazine, Pyrane, Piperidin, Piperazin, Morpholin, Perhydrothiazin, Dioxan sowie ihre teilhydrierten bzw. teilgesättigten Homologen, usw.. Oxo substituierte Heterocyclen sind hier und im folgenden insbesondere fünfgliedrige und sechsgliedrige Lactone und Lactame, wie z.B. Butyrolactone, Valerolactone, Butyrolactam, Valerolactam, aber auch bicyclische Systeme wie Camphan.

Die den Verbindungen der Formel I zugrundeliegenden Grundkörper (OH-Gruppe in 5-Position) sind aus der US-4,468,390 bekannt. Milbemycin-D-Halbester, deren 5-OH-Gruppe mit einer zweibasigen Säure verestert ist, werden in der EP-102,721 beschrieben. Ferner kennt man Milbemycin-Kohlensäureester (Oxycarbonyloxygruppe in 5-Position) aus der EP-142,969. Diese Verbindungen des Standes der Technik zeigen jedoch deutliche Schwächen (vgl. die weiter hinten beschriebenen biologischen Versuche) bei der Bekämpfung von endoparasitären Würmern bei Warmblüter.

Im Umfang der Formel I sind folgende interessante Untergruppen zu nennen:

a) Verbindungen der Formel I, worin R für Methyl, Ethyl, Isopropyl oder sek.-Butyl steht;

$R_1$ für Wasserstoff, Fluor oder Methyl steht:

$R_2$ eine der Gruppen

$$-\left[X-\underset{\underset{O}{\|}}{C}-CH_2\right]_2-R_3 \quad \text{oder} \quad -X-\underset{\underset{O}{\|}}{C}-R_4 \quad \text{repräsentiert;}$$

wobei X für Sauerstoff oder Schwefel steht;

$R_3$ für Halogen steht; und $R_4$ für Wasserstoff, $C_1$-$C_4$-Alkoxy, $C_3$-$C_6$-Cycloalkoxy, $C_2$-$C_6$-Alkenyloxy, unsubstituiertes oder durch Halogen, $C_1$-$C_4$-Alkoxy, Hydroxy und/oder COOG substituiertes $C_1$-$C_6$-Alkyl, wobei G für Wasserstoff oder ein Alkalimetallkation steht, $C_3$-$C_6$-Cycloalkyl, unsubstituiertes oder durch Halogen substituiertes $C_2$-$C_6$-Alkenyl, unsubstituiertes oder durch Halogen substituiertes $C_2$-$C_6$-Alkinyl oder unsubstituiertes oder durch Halogen, Methyl, Methoxy, Nitro, Cyano und/oder $CF_3$ substituiertes Phenyl steht.

Innerhalb der Gruppe a) ist folgende Gruppierung speziell hervorzuheben:

b) Verbindungen der Formel I, worin R und $R_1$ wie unter a) definiert sind; $R_2$ eine der Gruppen

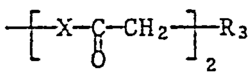 oder 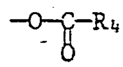 repräsentiert;

wobei $R_3$ für Fluor oder Chlor steht, $R_4$ für $C_1$-$C_4$-Alkoxy, $C_3$-$C_6$-Cycloalkoxy, $C_2$-$C_6$-Alkenyloxy, unsubstituiertes oder durch Fluor, Chlor, $C_1$-$C_4$-Alkoxy, Hydroxy oder COOH substituiertes $C_1$-$C_6$-Alkyl, $C_3$-$C_6$-Cycloalkyl, unsubstituiertes oder durch Fluor oder Chlor substituiertes $C_2$-$C_6$-Alkenyl, unsubstituiertes oder durch Fluor oder Chlor substituiertes $C_2$-$C_6$-Alkinyl oder unsubstituiertes oder durch Fluor, Chlor, Methyl, Methoxy, Nitro, Cyano und/oder $CF_3$ substituiertes Phenyl steht.

Hervorzuheben sind ferner:

c) Verbindungen der Formel I, worin R und $R_1$ wie in Gruppe a) definiert sind; $R_2$ die Gruppe

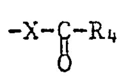 repräsentiert, wobei

X für Sauerstoff oder Schwefel steht; $R_4$ einen unsubstituierten oder

durch Oxo und/oder ein- bis dreifach durch Halogen, $C_1$-$C_4$-Alkyl, $C_1$-$C_4$-Alkoxy und/oder $C_1$-$C_4$-Haloalkyl substituierten fünf-oder sechsgliedrigen, ungesättigten oder gesättigten heterocyclischen Ring mit ein- bis drei Heteroatomen ausgewählt aus der Reihe Stickstoff, Sauerstoff und Schwefel bedeutet.

Innerhalb der Untergruppe c) sind bevorzugt zu nennen:

d) Verbindungen der Formel I, worin R und $R_1$ wie in Gruppe a) definiert sind; $R_2$ die Gruppe

$$-O-\underset{\underset{O}{\|}}{C}-R_4 \qquad \text{repräsentiert,}$$

$R_4$ einen unsubstituierten oder durch Oxo und/oder ein- bis dreifach durch Halogen, $C_1$-$C_4$-Alkyl, $C_1$-$C_4$-Alkoxy und/oder $C_1$-$C_4$-Haloalkyl substituierten fünf-oder sechsgliedrigen, ungesättigten oder gesättigten heterocyclischen Ring mit ein- bis drei Heteroatomen ausgewählt aus der Reihe Stickstoff, Sauerstoff und Schwefel bedeutet, wobei der heterocyclische Ring ausgewählt ist aus der Gruppe von Furan, Thiophen, Pyrrol, Isoxazol, Isothiazol, Furazan, Imidazol, 1,2,4-Triazol, 1,2,3-Triazol, Pyrazol, Pyrrolin, Oxazol, Thiazol, Thiadiazole, Pyrazolin, Thiazolin, Pyrazolidin, Pyrrolidin, Oxazolidin, Thiazolidin, Oxadiazol, Imidazolin, Imidazolidin, Pyrazolidin, Tetrahydrofuran; und typische sechsgliedgrige heterocyclische Ringe sind Pyridin, Pyridazin, Pyrimidin, Pyrazin, Thiazin, Thiadiazine, Pyrane, Piperidin, Piperazin, Morpholin, Perhydrothiazin, Dioxan sowie ihre teilhydrierten bzw. teilgesättigten Homologen, Butyrolactone, Valerolactone, Butyrolactam, Valerolactam, oder Camphan.

Bevorzugte Einzelsubstanzen der Formel I sind:

5-O-Acetoxy-acetyl-milbemycin D

5-O-Acetoxy-acetyl-milbemycin $A_4$

5-O-Acetoxy-acetyl-milbemycin $A_3$

5-O-Acetoxy-acetyl-13-deoxy-22,23-dihydro-avermectin-Bla-aglycon

5-0-(3,4-Dihydro-2H-pyran-2-yl)carboxy-acetyl-milbemycin D
5-0-(3,4-Dihydro-2H-pyran-2-yl)carboxy-acetyl-milbemycin $A_4$
5-0-(3,4-Dihydro-2H-pyran-2-yl)carboxy-acetyl-milbemycin $A_3$
5-0-(5-Pyrrolidon-2-yl)carboxy-acetyl-milbemycin D
5-0-(5-Pyrrolidon-2-yl)carboxy-acetyl-milbemycin $A_4$
5-0-((S)-5-Pyrrolidon-2-yl)carboxy-acetyl-milbemycin $A_4$
5-0-((R)-5-Pyrrolidon-2-yl)carboxy-acetyl-milbemycin $A_4$
5-0-Camphanoyloxyacetyl-milbemycin $A_4$
5-0-((S)-2-Hydroxy-propionyloxy)acetyl-milbemycin D
5-0-Chloracetoxyacetyl-milbemycin D
5-0-Chloracetoxy-acetoxy-acetyl-milbemycin D
5-0-Chloracetoxy-acetoxy-acetoxy-acetyl-milbemycin D
5-0-Acetylthio-acetyl-milbemycin D.
5-0-Fluoracetoxy-acetyl-milbemycin $A_4$
5-0-Formyloxy-acetyl-milbemycin $A_4$
5-0-Benzoyloxy-acetyl-milbemycin $A_4$
5-0-Propionyloxy-acetyl-milbemycin $A_4$
5-0-Methoxyacetoxy-acetyl-milbemycin $A_4$
5-0-(Acetoxy)-fluoracetyl-milbemycin $A_4$
5-0-(2-Acetoxy)-propionyl-milbemycin $A_4$
5-0-(2-Acetoxy)-butanoyl-milbemycin $A_4$
5-0-Cyclopropyl-carbonyloxy-acetyl-milbemycin $A_4$
5-0-Methoxy-carbonyloxy-acetyl-milbemycin $A_4$
5-0-(3-Chlorbenzoyloxy)-acetyl-milbemycin D
5-0-(3-Chlorbenzoyloxy)-acetyl-milbemycin $A_4$
5-0-(3-Chlorbenzoyloxy)-acetyl-milbemycin $A_3$
und
5-0-((S)-2-Hydroxy-propionyloxy)acetyl-milbemycin $A_4$.

Die vorliegende Erfindung betrifft nicht nur die Verbindungen der Formel I, sondern gleichermassen das neue Verfahren zu deren Herstellung.

Verbindungen der nachstehenden Formel II sind aus der Literatur als Milbemycine bekannt:

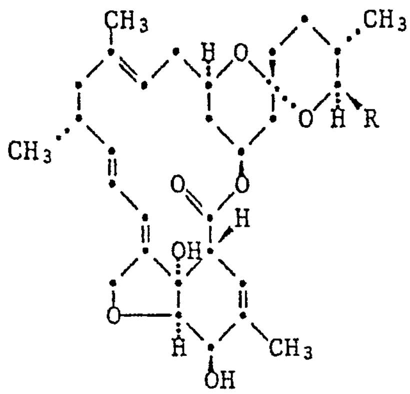

(II)

R = $CH_3$ Milbemycin $A_3$ aus US-PS 3,950,360

R = $C_2H_5$ Milbemycin $A_4$ aus US-PS 3,950,360

R = iso$C_3H_7$ Milbemycin D aus US-PS 4,346,171

R = sec.$C_4H_9$ 13-Desoxi-22,23-dihydro-C-076-Bla-aglycon, oder 13-Desoxi-22,23-dihydro-avermectin-Bla-aglykon aus US-PS 4,173,571.

Verbindungen, worin R sek.Butyl darstellt, sollen hier und im folgenden gleichfalls zu den Milbemycin-Derivaten gerechnet werden, obwohl sie nach der üblichen Systematik von Avermectin-Derivaten abgeleitet sind. Avermectin-Aglykone (mit einer OH-Gruppe in 13-Position) lassen sich jedoch gemäss US-PS 4,173,571 in Milbemycin-Homologe überführen.

Die Verbindungen der Formel I werden erfindungsgemäss folgendermassen hergestellt:

A) indem man

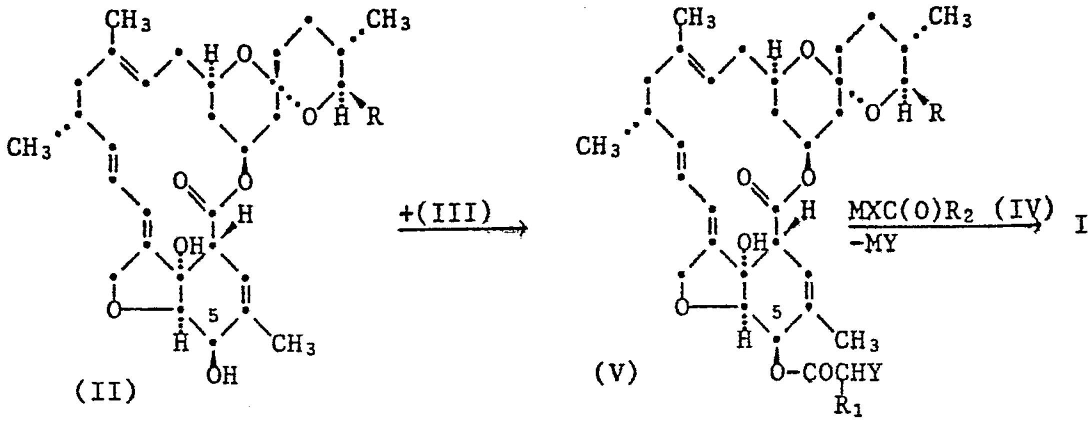


(II) (V)

das Milbemycin-Derivat der Formel II an der 5-OH Gruppe mit einem reaktionsfähigen Säurederivat der Formel III

$$Y-\underset{R_1}{CH}-CO-Z \qquad (III)$$

durch Veresterung in Zwischenprodukte der Formel V überführt und die Verbindung der Formel V mit einem Alkalisalz der (Thio)säure IV in das Endprodukt I überführt, wobei R, $R_1$, $R_2$ und X die unter Formel I angegebenen Bedeutungen haben und M ein Alkalimetallkation aus der Reihe Li, Na oder K, insbesondere Na oder K repräsentiert. Als reaktionsfähige Säurederivate dienen zur Acetylierung befähigte Verbindungen wie entsprechend substituierte Acetylhalogenide oder Acetanhydride oder die substituierte Essigsäure selbst, die allesamt in 5-Position den Rest

$$Y-\underset{R_1}{CH}-CO-$$

einführen. Z bedeutet somit Halogen, bevorzugt Chlor oder Brom, -OH oder die halbe Sauerstoff-Funktion im Säureanhydrid. Y steht für Halogen, bevorzugt Chlor, Brom oder Jod für Azido oder für eine andere nucleophil leicht ersetzbare Abgangsgruppe wie z.B. einen Sulfonsäurerest, vorzugsweise Mesylat oder Tosylat. Als Verbindungen der Formel III seien insbesondere hier genannt Chloracetylchlorid,

Bromacetylbromid, Azidoacetylchlorid, Chloracetanhydrid, 2-Mesylessigsäurechlorid. Im Falle der Säurehalogenide und Säureanhydride erfolgt die Umsetzung vorzugsweise in einem inerten, hydroxylgruppenfreien Lösungsmittel in Anwesenheit einer organischen Base, wie z.B. Pyridin, 4-Dimethylaminopyridin, Lutidin, Collidin, Trialkylamin, N-Dialkylanilin, oder bicyclische, nicht nucleophile Basen wie z.B. 1,4-Diazabicyclo[2.2.2]octan (DABCO), 1,5-Diazabicyclo[4.3.0]-non-5-en (DBN) oder 1,8-Diazabicyclo[5.4.0]undec-7-en (1,5-5) (DBU). Die Reaktion wird im allgemeinen bei Temperaturen von -30° bis +70°C, vorzugsweise von -10° bis +50°C durchgeführt. Man arbeitet dabei zweckmässigerweise in Gegenwart eines reaktionsinerten Lösungsmittels oder Lösungsmittelgemisches. Es eignen sich hierfür z.B. aliphatische und aromatische Kohlenwasserstoffe wie Benzol, Toluol, Xylole, Petrolether, Hexan; halogenierte Kohlenwasserstoffe wie Chlorbenzol, Methylenchlorid, Ethylenchlorid, Chloroform, Tetrachlorkohlenstoff, Tetrachlorethylen; Ether und etherartige Verbindungen wie Dialkylether (Diethylether, Diisopropylether, tert.-Butylmethylether usw.), Anisol, Dioxan, Tetrahydrofuran; Nitrile wie Acetonitril, Propionitril; Ester wie Ethylacetat (Essigsäureethylester), Propylacetat oder Butylacetat; Ketone wie Aceton, Diethylketon, Methylethylketon; Verbindungen wie Dimethylsulfoxid (DMSO), Dimethylformamid (DMF) und Gemische solcher Lösungsmittel untereinander. Man kann die Reaktion aber auch im Ueberschuss einer der oben genannten Basen durchführen.

Stellt die Verbindung der Formel III eine Carbonsäure dar (Z = OH), so wird die Veresterungsreaktion II mit III vorteilhafterweise in Gegenwart für Veresterungen üblicher, wasserabspaltenden Reagenzien durchgeführt, wie z.B. in Anwesenheit eines Carbodiimids [Dicyclohexylcarbodiimid (DCC)] oder eines 1-Alkyl-2-halogen-pyridiniumsalzes [1-Methyl-2-chlorpyridiniumjodid]. Diese Reaktion wird vorzugsweise in einem der obengenannten, reaktionsinerten Lösungsmittel und bei Temperaturen von -30° bis +70°C, vorzugsweise -10° bis +50°C durchgeführt. Man arbeitet bevorzugt in Gegenwart einer Base wie z.B. in Gegenwart eines organischen Amins z.B. eines Trialkylamins (Trimethylamin, Triethylamin, Tripropylamin, Diisopropylethylamin usw.), eines Pyridins (Pyridin selbst, 4-Dimethylaminopyridin, 4-Pyrrolidylaminopyridin usw.), eines Morpholins (N-Methylmorpholin), eines N,N-Dialkylanilins (N,N-Dimethylanilin, N-Methyl-N-ethylanilin) usw.

Die Reaktion von V zu I kann in Gegenwart einer der oben genannten Basen und/oder eines Katalysators durchgeführt werden. Als Base kann auch überschüssiges Alkalisalz der Formel V oder eine anorganische Base wie ein Alkali- oder Erdalkalicarbonat oder -hydrogencarbonat, wie $Na_2CO_3$, $K_2CO_3$, $MgCO_3$, $NaHCO_3$, $KHCO_3$ usw. dienen. Geeignete Katalysatoren sind Alkalijodide, insbesondere NaJ oder KJ. Diese Umsetzung wird bevorzugt in aprotischen polaren Lösungsmitteln wie z.B. Dimethylsulfoxid, Dimethylformamid, Dimethylacetamid, Acetonitril, Benzonitril oder Hexamethylphosphortriamid, durchgeführt. Geeignete Reaktionstemperaturen liegen im Bereich von ±0° bis +150°C, vorzugsweise +20° bis +120°C.

Man kann die erfindungsgemässen Verbindungen der Formel I auch in einer modifizierten Reaktion nach

B: herstellen, indem man ein Milbemycin-Derivat der Formel II an der 5-OH-Gruppe mit einem reaktionsfähigen Säurederivat der Formel VI,

$$Z-\underset{O}{\underset{\|}{C}}-\underset{R_1}{\underset{|}{C}}H-R_2 \qquad \text{(VI)},$$

worin Z die unter Formel III angebenen Bedeutungen hat, durch Veresterung direkt in das Endprodukt I überführt. Für diese Veresterungsreaktion gelten die bei der Reaktion von (II + III ⟶ V) genannten Reaktionsbedingungen.

Während Variante B die direkte Veresterung darstellt, handelt es sich bei Variante A lediglich um die Durchführung der gleichen Reaktion jedoch in zwei Teilschritten.

Die Verbindung der Formel VI sind bekannt oder lassen sich analog zu bekannten Vertretern herstellen, z.B. gemäss folgendem Schema

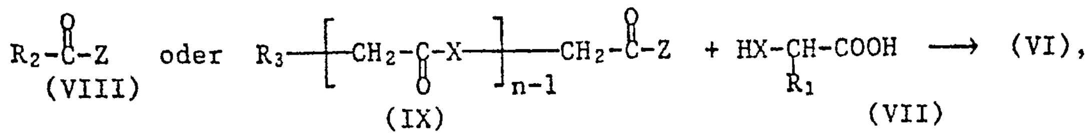
(VIII) oder (IX) (VII) (VI),

wobei die Substituenten die weiter oben angegebenen Bedeutungen haben. Dabei wird z.B. eine Säure der Formel VII (wie z.B. Glykolsäure, Milchsäure oder Thioglykolsäure) mit einem reaktionsfähigen Säurederivat der Formel VIII oder der Formel IX durch Veresterung unter den bereits oben geschilderten Reaktionsbedingungen in eine Verbindung der Formel VI, worin Z für OH steht, überführt. Die auf diese Weise erhaltene Säure der Formel VI (Z = OH) kann entweder direkt für die Umsetzung von II zu I verwendet werden oder zuerst, auf eine der gebräuchlichen Methoden, z.B. Umwandlung der Säure ins Säurechlorid mit Thionylchlorid, in eines ihrer reaktionsfähigen Säurederivate (z.B. ihr Säurechlorid) der Formel VI überführt werden. Unter den reaktionsfähigen Säurederivaten VI sind insbesondere jene bevozugt, worin Z für Halogen, vorzugsweise Chlor oder Brom oder für die halbe Säurefunktion (ein Anhydrid bildend) steht. Typische Vertreter der Formel VI sind:

O-Acetylglykolsäurebromid
2-Acetyloxy-acetylchlorid
O-Propionylglykolsäure und deren Chlorid sowie Bromid
2-Thioacetylessigsäure und deren Chlorid sowie Bromid
2-Acetoxypropionsäurechlorid
Dichloracetylglykolsäurechlorid
Trifluoracetylglykolsäurechlorid
2-Benzoyloxy-acetylchlorid.

Die Verbindungen der Formeln III, IV, VII, VIII und IX sind allgemein bekannt oder lassen sich analog zu ihren bekannten Vertretern herstellen.

Das beschriebene Verfahren zur Herstellung der Verbindungen der Formel I ist in allen Teilschritten ein Bestandteil vorliegender

Erfindung.

Die Verbindungen der Formel I eignen sich ausgezeichnet zur Bekämpfung von Schädlingen an Tieren und Pflanzen, darunter tierparasitären Ekto-Parasiten. Zu letzteren zählen unter der Ordnung Acarina insbesondere Schädlinge der Familien Ixodidae, Dermanyssidae, Sarcoptidae, Psoroptidae; die Ordnungen Mallophaga; Siphonaptera, Anoplura (z.B. Familie der Haemotopinidae); unter der Ordnung Diptera insbesondere Schädlinge der Familien Muscidae, Calliphoridae, Oestridae, Tabanidae, Hippoboscidae, Gastrophilidae.

Die Verbindungen I sind auch einsetzbar gegen Hygiene-Schädlinge, insbesondere der Ordnungen Diptera mit den Familien Sarcophagidae, Anophilidae, Culicidae; der Ordnung Orthoptera, der Ordnung Dictyoptera (z.B. Familie Blattidae) und der Ordnung Hymenoptera (z.B. Familie Formicidae).

Die Verbindungen I besitzen auch nachhaltige Wirksamkeit bei pflanzenparasitären Milben und Insekten. Bei Spinnmilben der Ordnung Acarina sind sie wirksam gegen Eier, Nymphen und Adulte von Tetranychidae (Tetranychus spp. und Panonychus spp.).

Hohe Aktivität besitzen sie bei den saugenden Insekten der Ordnung Homoptera, insbesondere gegen Schädlinge der Familien Aphididae, Delphacidae, Cicadellidae, Psyllidae, Loccidae, Diaspididae und Eriophydidae (z.B. die Rostmilbe auf Citrusfrüchten): Der Ordnungen Hemiptera; Heteroptera und Thysanoptera; sowie bei den pflanzenfressenden Insekten der Ordnungen Lepidoptera; Coleoptera; Diptera und Orthoptera.

Sie sind ebenfalls als Bodeninsektizid gegen Schädlinge im Erdboden geeignet.

Die Verbindungen der Formel I sind daher gegen alle Entwicklungsstadien saugender und fressender Insekten an Kulturen wie Getreide, Baumwolle, Reis, Mais, Soja, Kartoffeln, Gemüse, Früchten, Tabak,

Hopfen, Citrus, Avocados und anderen wirksam.

Die Verbindungen der Formel I sind auch wirksam gegen Pflanzen-Nematoden der Arten Meloidogyne, Heterodera, Pratylenchus, Ditylenchus, Radopholus, Rizoglyphus und andere.

Besonders aber sind die Verbindungen gegen Helminthen wirksam, unter denen die endoparasitären Nematoden die Ursache schwerer Erkrankungen an Säugetieren und Geflügel sein können, z.B. an Schafen, Schweinen, Ziegen, Rindern, Pferden, Eseln, Hunden, Katzen, Meerschweinchen, Ziervögeln. Typische Nematoden dieser Indikation sind: Haemonchus, Trichostrongylus, Ostertagia, Nematodirus, Cooperia, Ascaris, Bunostonum, Oesophagostonum, Charbertia, Trichuris, Strongylus, Trichonema, Dictyocaulus, Capillaria, Heterakis, Toxocara, Ascaridia, Oxyuris, Ancylostoma, Uncinaria, Toxascaris und Parascaris. Der besondere Vorteil der Verbindungen der Formel I ist ihre Wirksamkeit gegen solche Parasiten, die gegen Wirkstoffe auf Benzimidazol-Basis resistent sind.

Gewisse Spezies der Arten Nematodirus, Cooperia und Oesophagostomum greifen den Intestinaltrakt des Wirtstiers an, während andere der Arten Haemonchus und Ostertagia im Magen und solche der Art Dictyocaulus im Lungengewebe parasitieren. Parasiten der Familien Filariidae und Setariidae finden sich im internen Zellgewebe und den Organen, z.B. dem Herzen, den Blutgefässen, den Lymphgefässen und dem subcutanen Gewebe. Hier ist vor allem der Herzwurm des Hundes, Dirofilaria immitis, zu nennen. Die Verbindungen der Formel I sind gegen diese Parasiten hoch wirksam.

Sie sind ferner zur Bekämpfung von humanpathogenen Parasiten geeignet, unter denen als typische, im Verdauungstrakt vorkommende Vertreter solche der Arten Ancylostoma, Necator, Ascaris, Strongyloides, Trichinella, Capillaria, Trichuris und Enterobius zu nennen sind. Wirksam sind die Verbindungen vorliegender Erfindung auch gegen Parasiten der Arten Wuchereria, Brugia, Onchocerca und Loa aus der Familie der Filariidae, die im Blut, im Gewebe und verschiedenen

Organen vorkommen, ferner gegen Dracunculus und Parasiten der Arten Strongyloides und Trichinella, die speziell den Gastro-Intestinalkanal infizieren.

Die Verbindungen der Formel I werden in unveränderter Form oder vorzugsweise zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

Die Verbindungen der Formel I werden bei Warmblütern in Aufwandmengen von 0,01 bis 10 mg/kg Körpergewicht angewendet, über geschlossenen Kultur-Anbauflächen, in Pferchen, Stallungen oder sonstigen Räumen in Mengen von 10 g bis 1000 g pro Hektar.

Die Formulierungen, d.h. die den Wirkstoff der Formel I enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Ether und Ester, wie Ethanol, Ethylenglykol, Ethylenglykolmonomethyl- oder -ethylether, Ketone wie Cyclohexanon, stark polare Lösungsmittel wie N-Methyl-2-pyrrolidon, Dimethylsulfoxid oder Dimethylformamid, sowie gegebenenfalls epoxidierte Pflanzenöle, wie epoxidiertes Kokosnussöl oder

Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes nichtionogene, kation-und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen seien die Alkali-, Erdalkali- oder gegebenenfalls substituierte Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Talgöl gewonnen werden können, genannt. Ferner sind auch die Fettsäure-methyl-taurinsalze zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2-Sulfonsäuregruppen und einen Fettsäurerest mit 8 bis 22 C-Atomen. Alkylarylsulfonate sind z.B. die Na-, Ca- oder Triäthanolaminsalze der Dodecylbenzolsulfonsäure, der Dibutylnaphthalinsulfonsäure oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Aethylenoxid-Adduktes oder Phospholipide in Frage.

Die in der Formulierungstechnik gebräuchlichen Tenside sind u.a. in folgender Publikation beschrieben:

"Mc Cutcheon's Detergents and Emulsifiers Annual"
MC Publishing Corp., Ridgewood, New Jersey, 1982.

Die pestiziden Zubereitungen enthalten in der Regel 0,01 bis 95 %, insbesondere 0,1 bis 80 %, Wirkstoff der Formel I, 5 bis 99,99 % eines festen oder flüssigen Zusatzstoffes und 0 bis 25 %, insbesondere 0,1 bis 25 %, eines Tensides.

Während als Handelsware eher konzentrierte Mittel bevorzugt werden, verwendet der Endverbraucher in der Regel verdünnte Mittel mit 1-10'000 ppm Wirkstoffgehalt.

Ein weiterer Gegenstand vorliegender Erfindung betrifft daher Schädlingsbekämpfungsmittel, die neben üblichen Trägerstoffen und/oder Verteilungsmitteln als mindestens einen Wirkstoff eine Verbindung der Formel I enthalten.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

## Herstellungsbeispiele

### α-Herstellung von Zwischenprodukten der Formel V.

#### Z-1. Herstellung von 5-O-Chloracetyl-milbemycin D.

Zu einer Lösung von 400 mg Milbemycin D in 10 ml Pyridin werden unter Eiskühlung (0-5°C) 168 mg Chloracetylchlorid mittels einer Injektionsspritze langsam zugetropft. Die gelblichtrübe Reaktionslösung wird nach 2 Stunden Rühren bei 0°C auf 100 ml eiskalte 1 N wässrige Chlorwasserstoffsäure gegossen und viermal mit je 20 ml Diethylether (=Ether) extrahiert. Die sorgfältig gewaschene und getrocknete Etherlösung ergibt nach dem Eindampfen im Vakuum ein amorphes, schaumiges Produkt, das durch Säulenchromatographie an Silicagel mit dem Laufmittel Methylenchlorid/Methanol (98:2) gereinigt wird. Man erhält 410 mg des Endprodukts als farblose, amorphe Masse.

$^1H$-NMR (300 MHz; CDCl; TMS = Tetramethylsilan) 5,32 (s) ($-CH_2-Cl$), 3,25 (schmales Multiplett) ($C_2H$; d.h. H-Signal in Position 2); Massenspektrum m/e: 632 ($M^+$; $C_{35}H_{49}ClO_8$).

#### Z-2. Herstellung von 5-O-Chloracetyl-milbemycin $A_4$ und von 5-O-Chloracetyl-milbemycin $A_3$

Analog zu Beispiel (Z-1.) erhält man aus 3,00 g eines Gemisches aus 70 % Milbemycin $A_4$ und 30 % Milbemycin $A_3$ mit 0,88 ml Chloracetylchlorid und 4,4 ml Pyridin in 25 ml Methylenchlorid nach chromatographischer Trennung an Kieselgel (n-Hexan/Diethylether 2:1) 1,72 g 5-O-Chloracetyl-milbemycin $A_4$ und 0,77 g 5-O-Chloracetyl-milbemycin $A_3$ mit folgenden NMR-Daten:

5-O-Chloracetyl-milbemycin $A_4$
$^1H$-NMR (250 MHz, $CDCl_3$)
3,06 ppm (dt, $J_d$ = 2, $J_t$ = 8 Hz) ($C_{25}H$)
4,11 ppm (d, J = 7) ($C_6H$)
4,18 ppm (s) ($-CH_2Cl$)
5,57 ppm (bd, J = 7) ($C_5H$)

5-O-Chloracetyl-milbemycin $A_3$
$^1H$-NMR (250 MHz, $CDCl_3$)
3,26 ppm (m) ($C_{25}H$)
4,10 ppm (d, J = 7) ($C_6H$)
4,16 ppm (s) ($-CH_2Cl$)
5,58 ppm (d, J = 7) ($C_5H$)

Z-3. Herstellung von 5-O-Bromacetyl-milbemycin $A_4$

Analog zur Vorschrift H-1 erhält man aus Milbemycin $A_4$ ($R=C_2H_5$) und Bromacetylbromid in Pyridin 5-O-Bromacetylmilbemycin $A_4$ als amorphes Festprodukt.

Z-4. Herstellung von 5-O-Jodacetyl-milbemycin D

150 mg 5-O-Chloracetyl-milbemycin D und 50 mg Kaliumjodid werden in 10 ml Aceton gelöst und 24 Stunden bei Raumtemperatur gerührt. Nach dem Verdünnen der Reaktionslösung mit der 5-fachen Menge Wasser wird viermal mit je 10 ml Diethylether extrahiert und die aus der Etherlösung gewonnene Rohsubstanz an Silicagel chromatographiert (siehe Z-1). Man erhält 80 mg Reinprodukt. Smp. 133-137°C.

Z-5. Herstellung von 5-O-Azidoacetyl-milbemycin D

Analog zur Vorschrift Z-4 erhält man aus 150 mg 5-O-Chloracetyl-milbemycin D und 35 mg Natriumazid ($NaN_3$) 92 mg 5-O-Azidoacetyl-milbemycin D.

Z-6. Herstellung von 5-O-(2-Chlor)-butanoyl-milbemycin D

Zu einer Lösung von 284 mg Milbemycin D und 156 mg 2-Chlorbuttersäure in 3 ml Tetrahydrofuran werden 1,5 ml Pyridin und 212 mg N,N-Dicyclohexylcarbodiimid gegeben. Die Reaktionsmischung

wird 12 Stunden bei Raumtemperatur gerührt und dann auf 50 ml eiskalte 1N wässrige Chlorwasserstoffsäure gegossen und zweimal mit je 50 ml Diethylether extrahiert. Die Etherphasen werden mit je 30 ml gesättigter, wässriger $NaHCO_3$-Lösung und gesättigter, wässriger NaCl-Lösung gewaschen, mit $MgSO_4$ getrocknet, filtriert und eingedampft. Das erhaltene Rohprodukt wird an Kieselgel mit Hexan/Essigsäureethylester 7:1 chromatographiert. Man erhält 282 mg 5-O-(2-Chlor-)butanoyl-milbemycin D.

$^1H$-NMR (250 MHz, $CDCl_3$):
3,08 ppm (bd, J = 9) ($C_{25}H$)
4,33 ppm (m) ($OC(O)CH(Cl)CH_2$)
5,55 ppm (d, J = 6) ($C_5H$).

Z-7. Herstellung von 5-O-Bromfluoracetyl-milbemycin $A_4$

Zu einer Lösung von 542 mg Milbemycin $A_4$, 79 µl Pyridin und 6 mg Dimethylaminopyridin in 4 ml Methylenchlorid werden bei 0°C innerhalb von 30 Minuten 8 ml einer 0,25 M Lösung von Bromfluoracetylchlorid in Benzol zugetropft. Das Reaktionsgemisch wird 30 Minuten bei 0°C und 3 Stunden bei 25°C gerührt und anschliessend aufgearbeitet. Chromatographie des Rohproduktes an Kieselgel mit Hexan/Essigsäureethylester ergibt 662 mg 5-O-Bromfluoracetyl-milbemycin $A_4$.

Massenspektrum: m/e: 680, 682 ($M^+$, $C_{34}H_{46}BrFO_8$)

Nach Art der vorstehenden Beispiele lassen sich auch folgende Zwischenpodukte der Formel V gemäss vorliegender Erfindung herstellen:

| Nr. | |
|---|---|
| Z7. | 5-O-Bromacetyl-milbemycin $A_3$ |
| Z8. | 5-O-Bromacetyl-milbemycin-D |
| Z9. | 5-O-Chloracetyl-milbemycin $A_4$ |
| Z10. | 5-O-Fluoracetyl-milbemycin D |
| Z11. | 5-O-Methylsulfonyloxy-acetyl-milbemycin D |
| Z12. | 5-O-p-Tosyloxy-acetyl-milbemycin D |

Z13. 5-O-Azidoacetyl-milbemycin $A_3$

Z14. 5-O-Azidoacetyl-milbemycin $A_4$

Z15. 5-O-Ethylsulfonyloxy-acetyl-milbemycin $A_4$

Z16. 5-O-Chloracetyl-13-desoxy-22,23-dihydro-avermectin-Bla-aglykon

Z17. 5-O-Bromacetyl-13-desoxy-22,23-dihydro-avermectin-Bla-aglykon

Z18. 5-O-Azidoacetyl-13-desoxy-22,23-dihydro-avermectin-Bla-aglykon

sowie die nachfolgenden Verbindungen der Formel V:

| Verb. Nr. | R | $R_1$ | Y |
|---|---|---|---|
| Z19 | $CH_3$ | $CH_3$ | Cl |
| Z20 | $CH_3$ | $C_2H_5$ | Cl |
| Z21 | $CH_3$ | H | Cl |
| Z22 | $CH_3$ | H | $OSO_2C_6H_4CH_3(4)$ |
| Z23 | $C_2H_5$ | H | $OSO_2C_6H_4CH_3(4)$ |
| Z24 | $C_2H_5$ | $CH_3$ | Cl |
| Z25 | $C_2H_5$ | H | $OSO_2CH_3$ |
| Z26 | $C_2H_5$ | $C_3H_7$-n | $OSO_2C_6H_4CH_3(4)$ |
| Z27 | $C_2H_5$ | $C_2H_5$ | $OSO_2C_6H_4CH_3(4)$ |
| Z28 | $C_2H_5$ | H | J |
| Z29 | $C_2H_5$ | $CH_3$ | Br |
| Z30 | $C_3H_7$-i | $CH_3$ | $OSO_2C_6H_4CH_3(4)$ |
| Z31 | $C_3H_7$-i | H | $OSO_2CH_3$ |
| Z32 | $C_4H_9$-s | H | $OSO_2CH_3$ |
| Z33 | $C_4H_9$-s | H | $OSO_2C_6H_4CH_3(4)$ |
| Z34 | $C_4H_9$-s | $C_2H_5$ | Cl |
| Z35 | $C_2H_5$ | F | Br |
| Z36 | $C_2H_5$ | H | $OSO_2C_6H_4CH_3(4)$ |
| Z37 | $C_2H_5$ | $CH_3$ | Br |
| Z38 | $C_2H_5$ | H | $OSO_2CH_3$ |

β) Herstellung von Verbindungen der Formel I

H1: Herstellung von 5-O-Acetoxy-acetyl-milbemycin D

Eine Lösung von 150 mg 5-O-Chloracetyl-milbemycin D, 39 mg Natriumacetat und 2 mg Natriumjodid in 3 ml Dimethylformamid (DMF) wird 4 Stunden bei 50°C und anschliessend 30 Minuten bei 90°C unter Argonatmosphäre gerührt und dann auf 50 ml Diethylether gegossen. Die erhaltene Lösung wird mit 20 ml Wasser und 20 ml gesättigter NaCl-Lösung gewaschen, über $MgSO_4$ getrocknet, filtriert und eingedampft. Das erhaltene Rohprodukt wird an Kieselgel mit Hexan/Essigsäureethylester (2:1) chromatographiert. Man erhält 100 mg 5-O-Acetoxy-acetyl-milbemycin D.

$^1H$-NMR (250 MHz, $CDCl_3$)
2,18 ppm (s) ($CH_3C(O)O$)
3,08 ppm (bd, J = 10) ($C_{25}H$)
4,08 ppm (d, J = 7) ($C_6H$)
4,69 ppm (d, J = 15) (OC(O)C$\underline{H}$HO)
4,77 ppm (d, J = 15) (OC(O)CH$\underline{H}$O)
5,61 ppm (d, J = 7) ($C_5H$)

H2: Herstellung von 5-O-Acetoxy-acetyl-milbemycin $A_4$

a) Analog zu Beispiel H1 erhält man aus 150 mg 5-O-Chloracetyl-milbemycin $A_4$, 30 mg Natriumacetat und 2 mg Natriumjodid in 3 ml DMF nach Chromatographie an Kieselgel mit Hexan/Essigsäureethylester (2:1) 78 mg 5-O-Acetoxy-acetyl-milbemycin $A_4$.

b) Zu einer Lösung von 50 mg Milbemycin $A_4$, 73 µl Pyridin und 1 mg Dimethylaminopyridin in 1 ml Methylenchlorid werden bei 0°C innerhalb von 10 Minuten 2 ml einer 0,09 M Lösung von Acetoxyacetylchlorid in Benzol gegeben. Nach 2 Stunden Rühren bei 0°C wird die Reaktion aufgearbeitet. Chromatographie des Rohproduktes analog a) ergibt 48 mg Produkt.

$^1H$-NMR (250 MHz, $CDCl_3$):
2,18 ppm (s) ($CH_3OCO$)
3,06 ppm (dt, $J_d$ = 2, $J_t$ = 8) ($C_{25}H$)
4,08 ppm (d, J = 5) ($C_6H$)
4,66 ppm (d, J = 15) ($OC(O)C\underline{H}HO$)
4,75 ppm (d, J = 15) ($OC(O)CH\underline{H}O$)
5,61 ppm (bd, J = 5) ($C_5H$)

H3: Herstellung von 5-O-Acetoxy-acetyl-milbemycin $A_3$

Analog zu Beispiel H1 erhält man aus 100 mg 5-O-Chloracetyl-milbemycin $A_3$, 20 mg Natriumacetat und 2 mg Natriumjodid in 3 ml DMF nach Chromatographie an Kieselgel mit Hexan/Essigsäureethylester 2:1 53 mg 5-O-Acetoxy-acetyl-milbemycin $A_3$.

$^1H$-NMR (250 MHz, $CDCl_3$):
3,27 ppm (m) ($C_{25}H$)
4,06 ppm (d, J = 6) ($C_6H$)
4,68 ppm (d, J = 15) ($OC(O)C\underline{H}HO$)
4,76 ppm (d, J = 15) ($OC(O)CH\underline{H}O$)
5,61 ppm (d, J = 6) ($C_5H$)

H4: Herstellung von 5-O-(3,4-Dihydro-2H-pyran-2-yl)carboxyacetyl-milbemycin D

Eine Lösung von 150 mg 5-O-Chloracetyl-milbemycin D, 73 mg (±)-3,4-Dihydro-2H-pyran-2-carbonsäure-natriumsalz und 2 mg Kaliumjodid in 3 ml DMF wird 12 Stunden unter Argonatmosphäre bei 90°C gerührt und anschliessend mit 50 ml Diethylether verdünnt. Die erhaltene Lösung wird mit 20 ml Wasser und 20 ml gesättigter NaCl-Lösung gewaschen, über $MgSO_4$ getrocknet, filtriert und eingedampft. Das erhaltene Rohprodukt ergibt nach Chromatographie an Kieselgel mit Hexan/Essigsäureethylester (6:1) 58 mg 5-O-(3,4-Dihydro-2H-pyran-2-yl)carboxy-acetyl-milbemycin D.

$^1H$-NMR (250 MHz, $CDCl_3$)
3,07 ppm (bd, J = 10) ($C_{25}H$)
4,04 ppm und 4,06 ppm (2d, je J = 7) ($C_6H$)

4,58 ppm (bd, J = 15) ($C_{27}$HH)
4,66 ppm (bd, J = 15) ($C_{27}$HH)
6,43 ppm (d, J = 7) (O-CH=CH)

H5: Herstellung von 5-O-(3,4-Dihydro-2H-pyran-2-yl)carboxy-acetyl-milbemycin $A_4$

Analog zu Beispiel H4 wird eine Lösung von 150 mg 5-O-Chloracetyl-milbemycin A, 55 mg (±)-3,4-Dihydro-2H-pyran-2-carbonsäure-natriumsalz und 2 mg Natriumjodid in 3 ml DMF 3 Stunden bei 60°C und 1 Stunde bei 80°C umgesetzt. Man erhält, nach Chromatographie an Kieselgel mit Hexan/Essigsäureethylester (4:1) 131 mg 5-O-(3,4-Dihydro-2H-pyran-2-yl)carboxy-acetyl-milbemycin $A_4$.

$^1$H-NMR (250 MHz, $CDCl_3$)
3,07 (bt, J = 8) ($C_{25}$H)
4,06 ppm und 4,07 ppm (2d, je J = 7) ($C_6$H)
4,58 ppm (bd, J = 15) ($C_{27}$HH)
4,66 ppm (bd, J = 15) ($C_{27}$HH)
6,44 ppm (d, J = 7) (O-CH=CH)

H6: Herstellung von 5-O-(3,4-Dihydro-2H-pyran-2-yl)carboxy-acetyl-milbemycin $A_3$

Analog zu Beispiel H4 erhält man aus 80 mg 5-O-Chloracetyl-milbemycin $A_3$ 58 mg 5-O-(3,4-Dihydro-2H-pyran-2-yl)carboxy-acetyl-milbemycin $A_3$.

$^1$H-NMR (250 MHz, $CDCl_3$)
3,26 ppm (m) $C_{25}$H)
4,05 ppm und 4,06 ppm (2d, je J = 7) ($C_6$H)
4,58 ppm (bd, J = 15) ($C_{27}$HH)
4,66 ppm (bd, J = 15) ($C_{27}$HH)
6,46 ppm (d, J = 7) (O-CH=CH)

H7: Herstellung von 5-O-(5-Pyrrolidon-2-yl)carboxy-acetyl-milbemycin D

Eine Lösung von 150 mg 5-O-Chloracetyl-milbemycin D, 107 mg DL-Pyroglutaminsäure-natriumsalz und 2 mg NaJ in 3 ml DMF wird 4 Stunden unter Argonatmosphäre bei 70°C gerührt und dann mit 50 ml Diethylether verdünnt. Die erhaltene Lösung wird mit 20 ml Wasser und 20 ml gesättigter NaCl-Lösung gewaschen, über $MgSO_4$ getrocknet, filtriert und eingeengt. Nach Chromatographie des Rohprodukts an Kieselgel mit Essigsäureethylester erhält man 117 mg 5-O-(5-Pyrrolidon-2-yl)carboxyl-acetyl-milbemycin D.

$^1$H-NMR (250 MHz, $CDCl_3$)
3,01 ppm (bd, J = 10) ($C_{25}H$)
3,98 ppm (d, J = 7) ($C_6H$)
5,56 ppm (bd, J = 6) ($C_5H$)

H8: Herstellung von 5-O-(5-Pyrrolidon-2-yl)carboxy-acetyl-milbemycin $A_4$

Analog zu Beispiel H7 erhält man aus 150 mg 5-O-Chloracetyl-milbemycin $A_4$ 141 mg 5-O-(5-Pyrrolidon-2-yl)carboxy-acetyl-milbemycin $A_4$.

$^1$H-NMR (250 MHz, $CDCl_3$)
3,07 ppm (bt, J = 9) ($C_{25}H$)
4,03 ppm (d, J = 6) ($C_6H$)
5,62 ppm (bd, J = 7) ($C_5H$)

H9: Herstellung von 5-O-((S)-5-Pyrrolidon-2-yl)carboxy-acetyl-milbemycin $A_4$)

Analog zu Beispiel H7 erhält man aus 100 mg 5-O-Chloracetyl-milbemycin $A_4$, 73 mg L-Pyroglutaminsäure-natriumsalz und 2 mg Natriumjodid in 3 ml DMF nach Chromatographie an Kieselgel mit Essigsäureethylester 64 mg 5-O-((S)-5-Pyrrolidon-2-yl)carboxy-acetyl-milbemycin $A_4$.

$^1H$-NMR (250 MHz, $CDCl_3$)
3,08 ppm (dt, $J_d$ = 3, $J_t$ = 9) ($C_{25}H$)
4,03 ppm (d, J = 7) ($C_6H$)
4,50 ppm (bd, J = 15) ($C_{27}\underline{H}H$)
4,59 ppm (bd, J = 15) ($C_{27}H\underline{H}$)
4,76 ppm (d, J = 16) (OC(O)C$\underline{H}$HO)
4,84 ppm (d, J = 16) (OC(O)CH$\underline{H}$O)
5,62 ppm (bd, J = 7) ($C_5H$)

H10: Herstellung von 5-O-((R)-5-Pyrrolidon-2-yl)carboxy-acetyl-milbemycin $A_4$

Analog zu Beispiel H7 erhält man aus 100 mg 5-O-Chloracetyl-milbemycin $A_4$, 73 mg D-Pyroglutaminsäure Natriumsalz und 2 mg Natriumjodid in 3 ml DMF nach Chromatographie an Kieselgel mit Essigsäureethylester 91 mg 5-O-((R)-5-Pyrrolidon-2-yl)carboxyacetyl-milbemycin $A_4$.

$^1H$-NMR (250 MHz, $CDCl_3$)
3,08 ppm (dt, $J_d$ = 3, $J_t$ = 9) ($C_{25}H$)
4,04 ppm (d, J = 7) ($C_6H$)
4,59 ppm (bd, J = 15) ($C_{27}\underline{H}H$)
4,69 ppm (bd, J = 15) ($C_{27}H\underline{H}$)
4,76 ppm (d, J = 15) (OC(O)C$\underline{H}$HO)
4,84 ppm (d, J = 15) (OC(O)CH$\underline{H}$O)
5,63 ppm (d, J = 7) ($C_5H$)

H11: Herstellung von 5-O-Camphanoyloxy-acetyl-milbemycin $A_4$

Analog zu Beispiel H7 erhält man aus 100 mg 5-O-Chloracetyl-milbemycin $A_4$, 107 mg (-)-Camphansäure-natriumsalz und 2 mg Natriumjodid in 3 ml DMF nach Chromatographie an Kieselgel mit Hexan/Essigsäureethylester (2:1) 85 mg 5-O-Camphanoyloxyacetyl-milbemycin $A_4$.

$^1H$-NMR (250 MHz, $CDCl_3$)
3,08 ppm (dt, $J_d$ = 3, $J_t$ = 9) ($C_{25}H$)
4,06 ppm (d, J = 7) ($C_6H$)

4,59 ppm (bd, J = 15) ($C_{27}$HH)
4,68 ppm (bd, J = 15) ($C_{27}$HH)
4,87 ppm (bs) (OC(O)$CH_2$O)
5,63 ppm (bd, J = 7) ($C_5$H)

H12: Herstellung von 5-O-((S)-2-Hydroxy-propionyloxy)-acetyl-milbemycin D

Analog zu Beispiel H7 erhält man aus 100 mg 5-O-Chloracetyl-milbemycin D, 36 mg L-Milchsäure Natriumsalz und 2 µl Triethylamin in 3 ml DMF nach Chromatographie an Kieselgel mit Hexan/Essigsäureethylester (2:1) 77 mg 5-O-((S)-2-Hydroxy-propionyloxy)-acetyl-milbemycin D.

$^1$H-NMR (250 MHz, $CDCl_3$)
3,07 ppm (bd, J = 10) ($C_{25}$H)
4,06 ppm (d, J = 7) ($C_6$H)
4,78 ppm (d, J = 15) (OC(O)CHHO)
4,86 ppm (d, J = 15) (OC(O)CHHO)
5,60 ppm (bd, J = 7) ($C_5$H)

H13: Herstellung von 5-O-Chloracetoxy-acetyl-milbemycin D, 5-O-Chloracetoxy-acetoxy-acetyl-milbemycin D und 5-O-Chloracetoxy-acetoxy-acetoxy-acetyl-milbemycin D

Eine Lösung von 305 mg 5-O-Chloracetyl-milbemycin D, 168 mg Chloressigsäure Natriumsalz und 5 mg Natriumhydrogencarbonat in 5 ml Aceton wird analog zu Beispiel H7 umgesetzt. Die Chromatographie an Kieselgel mit Hexan/Essigsäureethylester (4:1) ergibt 45 mg 5-O-Chloracetoxyacetyl-milbemycin D.

$^1$H-NMR (250 MHz, $CDCl_3$)
3,08 ppm (bd, J = 10) ($C_{25}$H)
4,07 ppm (d, J = 7) ($C_6$H)
4,21 ppm (s) (-$CH_2$Cl)
4,78 ppm (d, J = 16) (OC(O)CHHO)
4,86 ppm (d, J = 16) (OC(O)CHHO)

Elution mit Hexan/Essigsäureethylester (3:1) ergibt 42 mg 5-O-Chloracetoxy-acetoxy-acetyl-milbemycin D

$^1$H-NMR (250 MHz, $CDCl_3$)
3,08 ppm (bd, J = 10) ($C_{25}H$)
4,07 ppm (d, J = 7) ($C_6H$)
4,20 ppm (s) ($-CH_2Cl$)
4,77 ppm (d, J = 15) ($C_5$-OC(O)C$\underline{H}$HO)
4,84 ppm (d, J = 15) ($C_5$-OC(O)CH$\underline{H}$O)
4,85 ppm (s) ($OC(O)CH_2O$)

Elution mit Hexan/Essigsäureethylester (2:1) ergibt 23 mg 5-O-Chloracetoxy-acetoxy-acetoxy-acetyl-milbemycin D

$^1$H-NMR (250 MHz, $CDCl_3$)
3,08 ppm (bd, J = 10) ($C_{25}H$)
4,06 ppm (d, J = 7) ($C_6H$)
4,21 ppm (s) ($-CH_2Cl$)
4,76 ppm (d, J = 15) ($C_5$-OC(O)C$\underline{H}$HO)
4,84 ppm (d, J = 15) ($C_5$-OC(O)CH$\underline{H}$O)
4,85 ppm (s) (2 x $OC(O)CH_2O$)

<u>H14: Herstellung von 5-O-Acethylthio-acetyl-milbemycin D</u>

Eine Lösung von 150 mg 5-O-Chloracetoxy-acetyl-milbemycin D, 54 mg Kaliumthioacetat und 10 µl Pyridin in 3 ml N,N-Dimethylacetamid wird 1 Stunde bei 70° gerührt und dann auf 50 ml Diethylether gegossen. Die erhaltene Lösung wird mit 20 ml Wasser und 20 ml gesättigter NaCl-Lösung gewaschen, über $MgSO_4$ getrocknet, filtriert und eingedampft. Chromatographie des Rohprodukts an Kieselgel mit Hexan/Essigsäureethylester (4:1) ergibt 121 mg 5-O-Thioloacetoxy-acetyl-milbemycin D.

$^1$H-NMR (250 MHz, $CDCl_3$)
2,37 ppm (s) ($CH_3(C(O)S)$)
3,08 ppm (d, J = 10) ($C_{25}H$)
3,76 ppm (d, J = 16) (OC(O)C$\underline{H}$HO)

3,86 ppm (d, J = 16) (OC(O)CHHO)
4,06 ppm (d, J = 7) ($C_6H$)

H15: Herstellung von 5-O-(Acetoxy)-fluoracetyl-milbemycin $A_4$

Eine Lösung von 150 mg 5-O-Bromfluoracetyl-milbemycin $A_4$, 27 mg Natriumacetat und 2 mg Natriumjodid in 2 ml DMF wird 1 Stunde bei 25°C und 3,5 Stunden bei 50°C gerührt und anschliessend aufgearbeitet. Chromatographie des Rohproduktes an Kieselgel mit Hexan/Essigsäureethylester ergibt 61 mg Produkt.

Massenspektrum: m/e: 660 ($M^+$, $C_{36}H_{49}FO_{10}$)
$^1$H-NMR (300MHz, $CDCl_3$):
3,07 ppm (dt, $J_d$ = 2, $J_t$ = 9) ($C_{25}H$)
6,51 ppm (d, J = 53) (OC(O)CHFO)

Gemäss den beschriebenen Arbeitsweisen lassen sich auch die nachfolgend aufgelisteten Verbindungen der Formel I herstellen, wobei die Auflistung beispielhaft zu verstehen ist und keinen limitierenden Charakter besitzt.

Tabelle 1: Verbindungen der Formel I (mit $R_1$ = H)

| Verb. Nr. | R | $R_2$ | physik. Daten bzw. Verweis auf Beispiel Nr. |
|---|---|---|---|
| 1.1 | $CH_3$ | $OC(O)CH_3$ | H3 |
| 1.2 | $CH_3$ | OC(O) 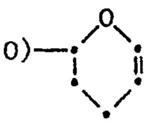 | H6 |
| 1.3 | $CH_3$ | $OC(O)CH_2Cl$ | |
| 1.4 | $CH_3$ | $OC(O)CH_2F$ | |
| 1.5 | $C_2H_5$ | $OC(O)CH_3$ | H2 |
| 1.6 | $C_2H_5$ | $OC(O)CH_2F$ | m/e:660($M^+$;$C_{36}H_{49}FO_{10}$) |
| 1.7 | $C_2H_5$ | $OC(O)CH_2Cl$ | m/e:676($M^+$;$C_{36}H_{49}ClO_{10}$) |
| 1.8 | $C_2H_5$ | $OC(O)CCl_3$ | m/e:746($M^+$;$C_{36}H_{47}Cl_3O_{10}$) |
| 1.9 | $C_2H_5$ | OC(O) 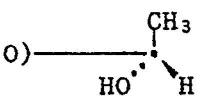 | m/e:672($M^+$;$C_{37}H_{52}O_{11}$) |
| 1.10 | $C_2H_5$ | OC(O)H | m/e:628($M^+$;$C_{35}H_{48}O_{10}$) |
| 1.11 | $C_2H_5$ | $OC(O)CH_2CH_3$ | m/e:656($M^+$;$C_{37}H_{52}O_{10}$) |
| 1.12 | $C_2H_5$ | $OC(O)C_4H_9$-t | m/e:648($M^+$;$C_{39}H_{56}O_{10}$) |
| 1.13 | $C_2H_5$ | $OC(O)C_3H_7$-i | m/e:670($M^+$;$C_{38}H_{54}O_{10}$) |
| 1.14 | $C_2H_5$ | OC(O)cyclopropyl | m/e:668($M^+$;$C_{38}H_{52}O_{10}$) |
| 1.15 | $C_2H_5$ | $OC(O)CH=CH_2$ | m/e:654($M^+$;$C_{37}H_{50}O_{10}$) |
| 1.16 | $C_2H_5$ | $OC(O)C\equiv CH$ | m/e:652($M^+$;$C_{37}H_{48}O_{10}$) |
| 1.17 | $C_2H_5$ | $OC(O)CH_2CH_2COOH$ | m/e:700($M^+$;$C_{38}H_{52}O_{12}$) |
| 1.18 | $C_2H_5$ | $OC(O)OCH_3$ | m/e:658($M^+$;$C_{36}H_{50}O_{11}$) |
| 1.19 | $C_2H_5$ | $OC(O)OCH=CH_2$ | m/e:670($M^+$;$C_{37}H_{50}O_{11}$) |
| 1.20 | $C_2H_5$ | $OC(O)CH_2CH_2CH_2F$ | |
| 1.21 | $C_2H_5$ | $OC(O)CH_2CH_2CH_2Cl$ | m/e:704($M^+$;$C_{38}H_{53}ClO_{10}$) |
| 1.22 | $C_2H_5$ | $OC(O)(CH_2)_7CH=CH(CH_2)_7CH_3$ | |
| 1.23 | $C_2H_5$ | $OC(O)C_6H_5$ | m/e:704($M^+$;$C_{41}H_{52}O_{10}$) |
| 1.24 | $C_2H_5$ | OC(O) 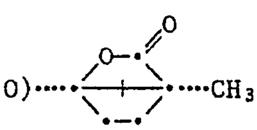 | H11 |
| 1.25 | $C_2H_5$ | $OC(O)C_6H_4(OH)(2)$ | m/e:720($M^+$;$C_{41}H_{52}O_{11}$) |

Tabelle 1: (Fortsetzung)

| Verb. Nr. | R | $R_2$ | physik. Daten bzw. Verweis auf Beispiel Nr. |
|---|---|---|---|
| 1.26 | $C_2H_5$ | $OC(O)C_6H_4Cl(3)$ | m/e:738($M^+$;$C_{41}H_{51}ClO_{10}$) |
| 1.27 | $C_2H_5$ | $OC(O)C_6H_4NO_2(4)$ | m/e:749($M^+$;$C_{41}H_{51}NO_{12}$) |
| 1.28 | $C_2H_5$ | OC(O) 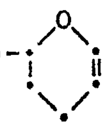 | H5 |
| 1.29 | $C_2H_5$ | OC(O) 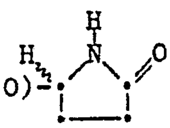 | H8 |
| 1.30 | $C_2H_5$ | OC(O) 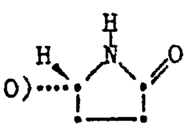 | H10 |
| 1.31 | $C_2H_5$ | OC(O) 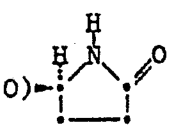 | H9 |
| 1.32 | $C_2H_5$ | OC(O) 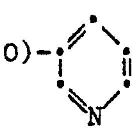 | m/e:705($M^+$;$C_{40}H_{51}NO_{10}$) |
| 1.33 | $C_2H_5$ | OC(O) 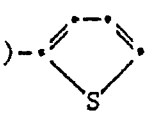 | m/e:710($M^+$;$C_{39}H_{50}O_{16}S$) |
| 1.34 | $C_3H_7$-i | $OC(O)CH_3$ | H1 |
| 1.35 | $C_3H_7$-i | $OC(O)CH_2Cl$ | H13 |
| 1.36 | $C_3H_7$-i | $OC(O)CH_2OC(O)CH_2Cl$ | H13 |
| 1.37 | $C_3H_7$-i | $OC(O)CH_2[OC(O)CH_2]_2Cl$ | H13 |
| 1.38 | $C_3H_7$-i | $OC(O)CH(OH)CH_3$ | H12 |
| 1.39 | $C_3H_7$-i | OC(O) 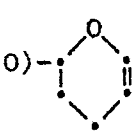 | H4 |
| 1.40 | $C_3H_7$-i | OC(O) 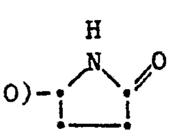 | H7 |

Tabelle 1: (Fortsetzung)

| Verb. Nr. | R | $R_2$ | physik. Daten bzw. Verweis auf Beispiel Nr. |
|---|---|---|---|
| 1.41 | $C_3H_7$-i | SC(O)$CH_3$ | H14 |
| 1.42 | $C_4H_9$-s | OC(O)$CH_3$ | |
| 1.43 | $C_4H_9$-s | OC(O)$CH_2Cl$ | |
| 1.44 | $C_4H_9$-s | OC(O)$C_6H_4NO_2$(3) | |
| 1.45 | $C_4H_9$-s | OC(O)- 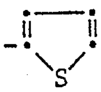 | |
| 1.46 | $C_2H_5$ | OC(O)$(CH_2)_{16}CH_3$ | m/e:866($M^+$;$C_{52}H_{82}O_{10}$) |
| 1.47 | $C_2H_5$ | OC(O)$C_6H_4F$(2) | m/e:722($M^+$;$C_{41}H_{51}FO_{10}$) |
| 1.48 | $C_2H_5$ | OC(O)$C_6H_3(NO_2)_2$(3,5) | m/e:794($M^+$;$C_{41}H_{50}N_2O_{14}$) |
| 1.49 | $C_2H_5$ | OC(O)-furan-2-yl | m/e:694($M^+$;$C_{39}H_{50}O_{11}$) |
| 1.50 | $C_2H_5$ | OC(O) 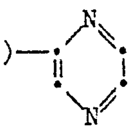 | m/e:706($M^+$;$C_{39}H_{50}N_2O_{10}$) |
| 1.51 | $C_2H_5$ | OC(O) 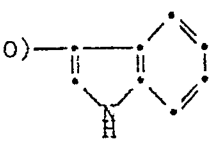 | m/e:743($M^+$;$C_{43}H_{53}NO_{10}$) |
| 1.52 | $C_2H_5$ | OC(O) 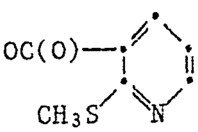 | |
| 1.53 | $C_2H_5$ | OC(O) 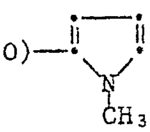 | |
| 1.54 | $C_2H_5$ | OC(O) 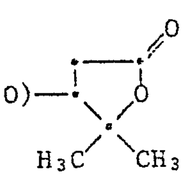 | |
| 1.55 | $C_2H_5$ | OC(O) 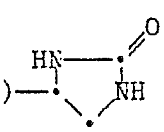 | |

Tabelle 1: (Fortsetzung)

| Verb. Nr. | R | $R_2$ | physik. Daten bzw. Verweis auf Beispiel Nr. |
|---|---|---|---|
| 1.56 | $C_2H_5$ | OC(O)— 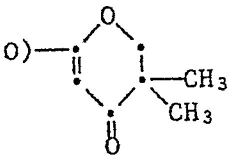 | |
| 1.57 | $C_2H_5$ | $OC(O)C_6H_4CF_3(4)$ | |
| 1.58 | $C_2H_5$ | $OC(O)CH_2CF_3$ | |
| 1.59 | $C_2H_5$ | $OCO-C_6H_4NO_2(3)$ | m/e:749($M^+$;$C_{41}H_{51}NO_{12}$) |
| 1.60 | $C_2H_5$ | $OCO-C_6H_4CF_3(3)$ | m/e:772($M^+$;$C_{42}H_{51}F_3O_{10}$) |
| 1.61 | $C_2H_5$ | $OCO-C_6H_4Br(3)$ | m/e:784/782($M^+$;$C_{41}H_{51}BrO_{10}$) |
| 1.62 | $C_2H_5$ | $OCO-C_6H_4F(3)$ | m/e:722($M^+$;$C_{41}H_{51}O_{10}F$) |
| 1.63 | $C_2H_5$ | $OCO-C_6H_4CH_3(3)$ | m/e:718($M^+$;$C_{42}H_{54}O_{10}$) |
| 1.64 | $C_2H_5$ | $OCO-C_6H_4OCH_3(3)$ | m/e:734($M^+$;$C_{42}H_{54}O_{11}$) |
| 1.65 | $C_2H_5$ | $OCOCH_2OH$ | m/e:658($M^+$;$C_{36}H_{50}O_{11}$) |
| 1.66 | $C_2H_5$ | $OCOCH_2OCOCH_3$ | m/e:700($M^+$;$C_{36}H_{52}O_{12}$) |

Tabelle 2: Verbindungen der Formel I (mit $R_2$ = $CH_3C(O)-$)

| Verb. Nr. | R | $R_1$ | physik. Daten bzw. Hinweis auf Beispiel Nr. |
|---|---|---|---|
| 2.1 | $CH_3$ | F | |
| 2.2 | $CH_3$ | $CH_3$ | |
| 2.3 | $C_2H_5$ | F | m/e:660($M^+$;$C_{36}H_{49}FO_{10}$) |
| 2.4 | $C_2H_5$ | $CH_3$ | |
| 2.5 | $C_2H_5$ | $C_2H_5$ | |
| 2.6 | $C_2H_5$ | $C_3H_7$-n | |
| 2.7 | $C_3H_7$-i | F | |
| 2.8 | $C_3H_7$-i | $CH_3$ | |
| 2.9 | $C_4H_9$-s | F | |
| 2.10 | $C_4H_9$-s | $CH_3$ | |

Formulierungsbeispiele für den Wirkstoff der Formel I

(% = Gewichtsprozent)

| Spritzpulver | a) | b) | c) |
|---|---|---|---|
| Wirkstoff aus den Tabellen 1 und 2 | 25 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyethylenglykol-ether (7-8 Mol AeO) | - | 2 % | - |
| Hochdisperse Kieselsäure | 5 % | 10 % | 10 % |
| Kaolin | 62 % | 27 % | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| Emulsions-Konzentrat | |
|---|---|
| Wirkstoff aus den Tabellen 1 und 2 | 10 % |
| Octylphenolpolyethylenglykolether (4-5 Mol AeO) | 3 % |
| Ca-Dodecylbenzolsulfonat | 3 % |
| Ricinusölpolyglykolether (36 Mol AeO) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

| Stäubemittel | a) | b) |
|---|---|---|
| Wirkstoff Nr. aus den Tabellen 1 und 2 | 5 % | 8 % |
| Talkum | 95 % | - |
| Kaolin | - | 92 % |

Man erhält anwendungsfertige Stäubemittel, indem der Wirkstoff mit dem Träger vermischt und auf einer geeigneten Mühle vermahlen wird.

Extruder Granulat

| | |
|---|---|
| Wirkstoff aus den Tabellen 1 und 2 | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschliessend im Luftstrom getrocknet.

Tabellen bzw. Boli

| | | |
|---|---|---|
| I | Ein Wirkstoff aus den Tabellen 1 und 2 | 33,0 % |
| | Methylcellulose | 0,80 % |
| | Kieselsäure hochdispers | 0,80 % |
| | Maisstärke | 8,40 % |

Methylcellulose in Wasser einrühren und quellen lassen; Kieselsäure in die Quellung einrühren und homogen suspendieren. Wirkstoff und Maisstärke mischen. In diese Mischung die wässrige Suspension einarbeiten und zu einem Teig kneten. Diese Masse durch ein Sieb (Maschenweite 12 M) granulieren und dann trocknen.

| | | |
|---|---|---|
| II | Milchzucker krist. | 22,50 % |
| | Maisstärke | 17,00 % |
| | mikrokrist. Cellulose | 16,50 % |
| | Magnesiumstearat | 1,00 % |

Alle 4 Hilfsstoffe gut mischen

Phasen I und II mischen und zu Tabletten oder Boli verpressen.

Falls die Verbindungen der Formel I bzw. entsprechende Mittel zur Bekämpfung von endoparasitären Nematoden, Cestoden und Trematoden bei Haus- und Nutztieren, wie Rindern, Schafen, Ziegen, Katzen und Hunden, verwendet werden, können sie den Tieren sowohl als Einzeldosis wie auch wiederholt verabreicht werden, wobei die einzelnen Gaben je nach Tierart vorzugsweise zwischen 0,1 und 10 mg pro kg Körpergewicht betragen. Durch eine protrahierte Verabreichung erzielt man in manchen Fällen eine bessere Wirkung oder man kann mit geringeren Gesamtdosen auskommen. Der Wirkstoff bzw. die ihn enthaltenden Mittel können auch dem Futter oder den Tränken zugesetzt werden. Das Fertigfutter enthält die Wirkstoffkombinationen vorzugsweise in einer Konzentration von 0,005 bis 0,1 Gew. %. Die Mittel können in Form von Lösungen, Emulsionen, Suspensionen, Pulver, Tabletten, Bolussen oder Kapseln peroral den Tieren verabreicht werden. Soweit die physikalischen und toxikologischen Eigenschaften von Lösungen oder Emulsionen dies zulassen, können die Verbindungen der Formel I bzw. die enthaltende Mittel an Tieren auch beispielsweise subcutan injiziert, intraruminal verabreicht oder mittels der Pour-on-Methode auf den Körper der Tiere appliziert werden. Ferner ist eine Verabreichung des Wirkstoffs an die Tiere auch durch Lecksteine (Salz) oder Molasse-Blöcke möglich.

Biologische Beispiele

In den nachfolgenden biologischen Untersuchungen wurden folgende Substanzen des Standes der Technik vergleichend mitgeprüft:

(A)

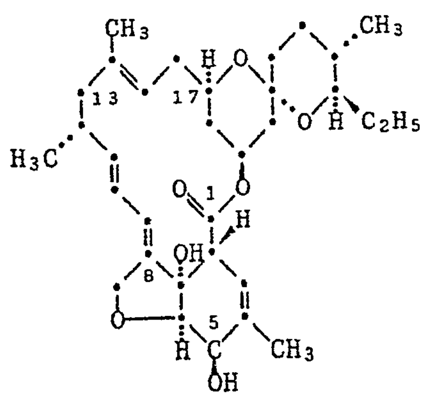

bekannt aus US-4,468,390

(B)

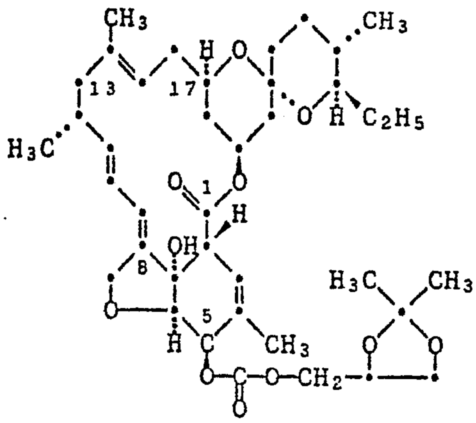

bekannt aus
EP-142,969

(C)

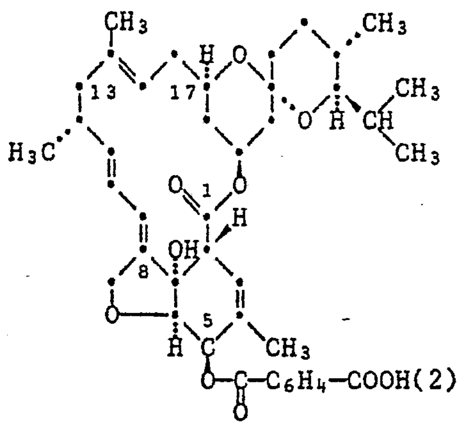

bekannt aus
EP-102,721

(D)

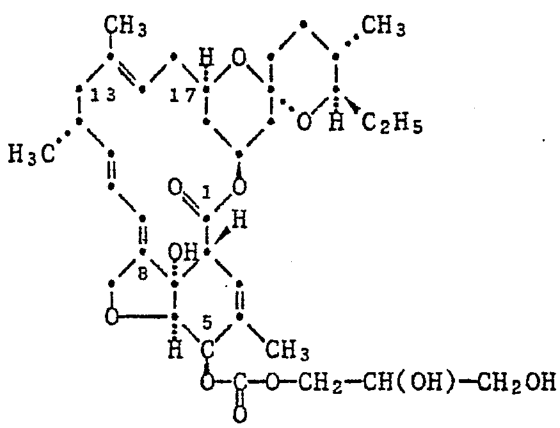

bekannt aus
EP-142,969

(E) 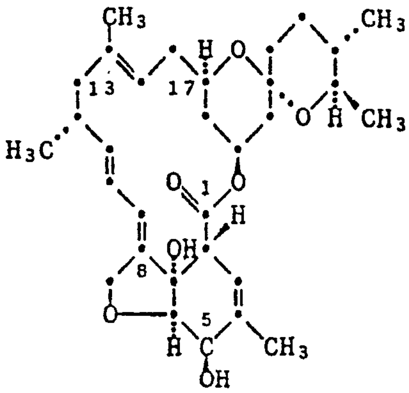 bekannt aus US-4,468,390

<u>B-1. Versuch an mit Nematoden (Haemonchus concortus und Trichostrongylus colubriformis) infizierten Schafen</u>

Der Wirkstoff wird als Suspension formuliert mit einer Magensonde oder durch Injektion in den Pansen eines Schafes gegeben, das mit Haemonchus concortus und Trychostrongylus colubriformis künstlich infiziert worden ist. Pro Dosis werden 1 bis 3 Tiere verwendet. Jedes Schaf wird nur einmal mit einer einzigen Dosis behandelt, und zwar wahlweise mit 0,5 g oder 0,1 mg/kg Körpergewicht. Die Evaluierung erfolgt durch Vergleich der Anzahl der vor und nach Behandlung im Kot der Schafe ausgeschiedenen Wurmeier.

Bei Schafen, die mit einer der Verbindungen der Formel I, wie z.B. mit Nr. 1.1, 1.2, 1.5 bis 1.19, 1.21, 1.23, 1.24, 1.25 bis 1.41, 1.46 bis 1.51, 1.59 bis 1.62, 1.65 oder 2.3 bei 0,5 mg/kg behandelt wurden fand man volle Wirkung; bei 0,1 mg/kg fand man eine Wirkung von 80 bis 100 %. Dagegen zeigten die Substanzen (A) bis (E) des Standes der Technik eine völlig unzureichende nematizide Wirkung, die zwischen 20 und 58 % lag. Als Vergleichsbasis dienten unbehandelte, aber infizierte Schafe. Die Versuchsauswertung erfolgte durch Auszählen der Wurmeier im Kot, wobei die vollständige Reduktion der Wurmeier eine 100%ige Wirkung verkörpert, während die mittlere Zahl von Eiern im Kot der Kontrolltiere die Null-Wirkung charakterisierte.

Patentansprüche für die Vertragsstaaten: DE, GB, FR, CH, LI, IT, NL, BE, SE, LU

1. Verbindungen der Formel I

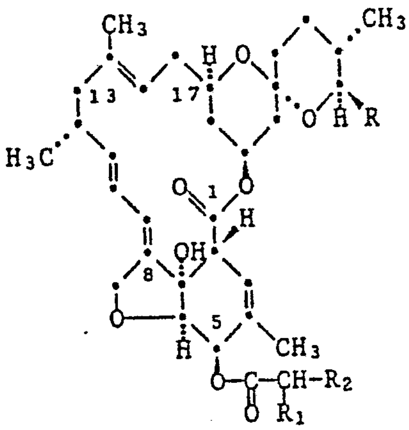

(I)

worin

R für Methyl, Ethyl, Isopropyl oder sek.-Butyl steht;

$R_1$ für Wasserstoff, Fluor oder $C_1$-$C_4$-Alkyl steht;

$R_2$ eine der Gruppen

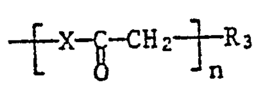 oder 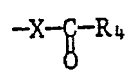 repräsentiert; wobei

n eine der Zahlen 2 bis 6 bedeutet;

X für Sauerstoff oder Schwefel steht ;

$R_3$ für Halogen steht; und

$R_4$ Wasserstoff, $C_1$-$C_{12}$-Alkoxy, $C_3$-$C_7$-Cycloalkoxy, $C_2$-$C_6$-Alkenyloxy, unsubstituiertes oder durch Halogen, $C_1$-$C_4$-Alkoxy, Hydroxy und/oder COOG substituiertes $C_1$-$C_{18}$-Alkyl, wobei G für Wasserstoff, ein Alkalimetall- oder Erdalkalimetallkation steht, unsubstituiertes oder durch Halogen und/oder $C_1$-$C_4$-Alkyl substituiertes $C_3$-$C_7$-Cycloalkyl, unsubstituiertes oder durch Halogen substituiertes $C_2$-$C_{18}$-Alkenyl, unsubstituiertes oder durch Halogen substituiertes $C_2$-$C_{18}$-Alkinyl, unsubstituiertes oder durch Halogen, $C_1$-$C_4$-Alkyl, $C_1$-$C_4$-Alkoxy, Nitro, Cyano und/oder $C_1$-$C_4$-Haloalkyl substituiertes Phenyl oder einen unsubstituierten oder durch Oxo und/oder ein- bis dreifach durch Halogen, $C_1$-$C_4$-Alkyl, $C_1$-$C_4$-Alkoxy und/oder $C_1$-$C_4$-Haloalkyl substituierten fünf-

oder sechsgliedrigen, ungesättigten oder gesättigten heterocyclischen Ring mit ein- bis drei Heteroatomen ausgewählt aus der Reihe Stickstoff, Sauerstoff und Schwefel bedeutet.

2. Verbindungen der Formel I nach Anspruch 1, worin R für Methyl, Ethyl, Isopropyl oder sek.-Butyl steht;
$R_1$ für Wasserstoff, Fluor oder Methyl steht:
$R_2$ eine der Gruppen

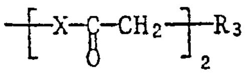 oder 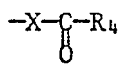 repräsentiert;

wobei X für Sauerstoff oder Schwefel steht;
$R_3$ für Halogen steht; und $R_4$ für Wasserstoff, $C_1$-$C_4$-Alkoxy, $C_3$-$C_6$-Cycloalkoxy, $C_2$-$C_6$-Alkenyloxy, unsubstituiertes oder durch Halogen, $C_1$-$C_4$-Alkoxy, Hydroxy und/oder COOG substituiertes $C_1$-$C_6$-Alkyl, wobei G für Wasserstoff oder ein Alkalimetallkation steht, $C_3$-$C_6$-Cycloalkyl, unsubstituiertes oder durch Halogen substituiertes $C_2$-$C_6$-Alkenyl, unsubstituiertes oder durch Halogen substituiertes $C_2$-$C_6$-Alkinyl oder unsubstituiertes oder durch Halogen, Methyl, Methoxy, Nitro, Cyano und/oder $CF_3$ substituiertes Phenyl steht.

3. Verbindungen der Formel I nach Anspruch 2, worin R und $R_1$ wie in Anspruch 2 definiert sind; $R_2$ eine der Gruppen

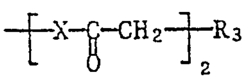 oder 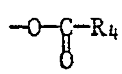 repräsentiert;

wobei $R_3$ für Fluor oder Chlor steht, $R_4$ für $C_1$-$C_4$-Alkoxy, $C_3$-$C_6$-Cyclolkoxy, $C_2$-$C_6$-Alkenyloxy, unsubstituiertes oder durch Fluor, Chlor, $C_1$-$C_4$-Alkoxy, Hydroxy oder COOH substituiertes $C_1$-$C_6$-Alkyl, $C_3$-$C_6$-Cycloalkyl, unsubstituiertes oder durch Fluor oder Chlor substituiertes $C_2$-$C_6$-Alkenyl, unsubstituiertes oder durch Fluor oder Chlor substituiertes $C_2$-$C_6$-Alkinyl oder unsubstituiertes oder durch Fluor, Chlor, Methyl, Methoxy, Nitro, Cyano und/oder $CF_3$ substituiertes Phenyl steht.

4. Verbindungen der Formel I nach Anspruch 1, worin R und $R_1$ wie in Anspruch 1 definiert sind; $R_2$ die Gruppe

$-X-\underset{\underset{O}{\|}}{C}-R_4$ repräsentiert, wobei

X für Sauerstoff oder Schwefel steht; $R_4$ einen unsubstituierten oder durch Oxo und/oder ein- bis dreifach durch Halogen, $C_1$-$C_4$-Alkyl, $C_1$-$C_4$-Alkoxy und/oder $C_1$-$C_4$-Haloalkyl substituierten fünf-oder sechsgliedrigen, ungesättigten oder gesättigten heterocyclischen Ring mit ein- bis drei Heteroatomen ausgewählt aus der Reihe Stickstoff, Sauerstoff und Schwefel bedeutet.

5. Verbindungen der Formel I nach Anspruch 4, worin R und $R_1$ wie in Anspruch 1 definiert sind; $R_2$ die Gruppe

$-O-\underset{\underset{O}{\|}}{C}-R_4$ repräsentiert,

$R_4$ einen unsubstituierten oder durch Oxo und/oder ein- bis dreifach durch Halogen, $C_1$-$C_4$-Alkyl, $C_1$-$C_4$-Alkoxy und/oder $C_1$-$C_4$-Haloalkyl substituierten fünf-oder sechsgliedrigen, ungesättigten oder gesättigten heterocyclischen Ring mit ein- bis drei Heteroatomen ausgewählt aus der Reihe Stickstoff, Sauerstoff und Schwefel bedeutet, wobei der heterocyclische Ring ausgewählt ist aus der Gruppe von Furan, Thiophen, Pyrrol, Isoxazol, Isothiazol, Furazan, Imidazol, 1,2,4-Triazol, 1,2,3-Triazol, Pyrazol, Pyrrolin, Oxazol, Thiazol, Thiadiazole, Pyrazolin, Thiazolin, Pyrazolidin, Pyrrolidin, Oxazolidin, Thiazolidin, Oxadiazol, Imidazolin, Imidazolidin, Pyrazolidin, Tetrahydrofuran; und typische sechsgliedgrige heterocyclische Ringe sind Pyridin, Pyridazin, Pyrimidin, Pyrazin, Thiazin, Thiadiazine, Pyrane, Piperidin, Piperazin, Morpholin, Perhydrothiazin, Dioxan sowie ihre teilhydrierten bzw. teilgesättigten Homologen, Bytrolactone, Valerolactone, Butyrolactam, Valerolactam, oder Camphan.

6. Eine Verbindung der Formel I nach Anspruch 1, ausgewählt aus der Reihe:

5-O-Acetoxy-acetyl-milbemycin D
5-O-Acetoxy-acetyl-milbemycin $A_4$
5-O-Acetoxy-acetyl-milbemycin $A_3$
5-O-Acetoxy-acetyl-13-deoxy-22,23-dihydro-avermectin-Bla-aglycon
5-O-(3,4-Dihydro-2H-pyran-2-yl)carboxy-acetyl-milbemycin D
5-O-(3,4-Dihydro-2H-pyran-2-yl)carboxy-acetyl-milbemycin $A_4$
5-O-(3,4-Dihydro-2H-pyran-2-yl)carboxy-acetyl-milbemycin $A_3$
5-O-(5-Pyrrolidon-2-yl)carboxy-acetyl-milbemycin D
5-O-(5-Pyrrolidon-2-yl)carboxy-acetyl-milbemycin $A_4$
5-O-((S)-5-Pyrrolidon-2-yl)carboxy-acetyl-milbemycin $A_4$
5-O-((R)-5-Pyrrolidon-2-yl)carboxy-acetyl-milbemycin $A_4$
5-O-Camphanoyloxyacetyl-milbemycin $A_4$
5-O-((S)-2-Hydroxy-propionyloxy)acetyl-milbemycin D
5-O-Chloracetoxyacetyl-milbemycin D
5-O-Chloracetoxy-acetoxy-acetyl-milbemycin D
5-O-Chloracetoxy-acetoxy-acetoxy-acetyl-milbemycin D
5-O-Acetylthio-acetyl-milbemycin D.
5-O-Fluoracetoxy-acetyl-milbemycin $A_4$
5-O-Formyloxy-acetyl-milbemycin $A_4$
5-O-Benzoyloxy-acetyl-milbemycin $A_4$
5-O-Propionyloxy-acetyl-milbemycin $A_4$
5-O-Methoxyacetoxy-acetyl-milbemycin $A_4$
5-O-(Acetoxy)-fluoracetyl-milbemycin $A_4$
5-O-(2-Acetoxy)-propionyl-milbemycin $A_4$
5-O-(2-Acetoxy)-butanoyl-milbemycin $A_4$
5-O-Cyclopropyl-carbonyloxy-acetyl-milbemycin $A_4$
5-O-Methoxy-carbonyloxy-acetyl-milbemycin $A_4$
5-O-(3-Chlorbenzoyloxy)-acetyl-milbemycin D
5-O-(3-Chlorbenzoyloxy)-acetyl-milbemycin $A_4$
5-O-(3-Chlorbenzoyloxy)-acetyl-milbemycin $A_3$
und
5-O-((S)-2-Hydroxy-propionyloxy)acetyl-milbemycin $A_4$.

7. Verfahren zur Herstellung von Verbindungen der Formel I

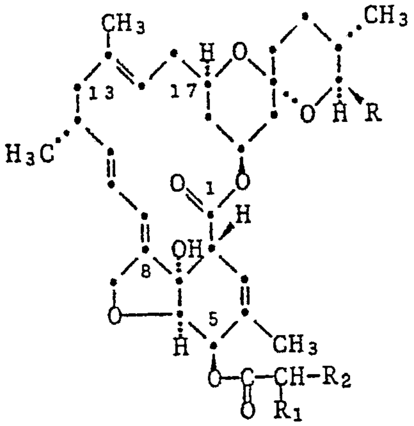

(I)

worin

R für Methyl, Ethyl, Isopropyl oder sek.-Butyl steht;

$R_1$ für Wasserstoff, Fluor oder $C_1$-$C_4$-Alkyl steht;

$R_2$ eine der Gruppen

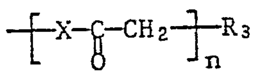 oder 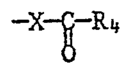 repräsentiert; wobei

n eine der Zahlen 2 bis 6 bedeutet;

X für Sauerstoff oder Schwefel steht ;

$R_3$ für Halogen steht; und

$R_4$ Wasserstoff, $C_1$-$C_{12}$-Alkoxy, $C_3$-$C_7$-Cycloalkoxy, $C_2$-$C_6$-Alkenyloxy, unsubstituiertes oder durch Halogen, $C_1$-$C_4$-Alkoxy, Hydroxy und/oder COOG substituiertes $C_1$-$C_{18}$-Alkyl, wobei G für Wasserstoff, ein Alkalimetall- oder Erdalkalimetallkation steht, unsubstituiertes oder durch Halogen und/oder $C_1$-$C_4$-Alkyl substituiertes $C_3$-$C_7$-Cycloalkyl, unsubstituiertes oder durch Halogen substituiertes $C_2$-$C_{18}$-Alkenyl, unsubstituiertes oder durch Halogen substituiertes $C_2$-$C_{18}$-Alkinyl, unsubstituiertes oder durch Halogen, $C_1$-$C_4$-Alkyl, $C_1$-$C_4$-Alkoxy, Nitro, Cyano und/oder $C_1$-$C_4$-Haloalkyl substituiertes Phenyl oder einen unsubstituierten oder durch Oxo und/oder ein- bis dreifach durch Halogen, $C_1$-$C_4$-Alkyl, $C_1$-$C_4$-Alkoxy und/oder $C_1$-$C_4$-Haloalkyl substituierten fünf- oder sechsgliedrigen, ungesättigten oder gesättigten heterocyclischen Ring mit ein- bis drei Heteroatomen ausgewählt aus der Reihe Stickstoff, Sauerstoff und Schwefel bedeutet, dadurch gekennzeichnet, dass man eine Verbindung der Formel II

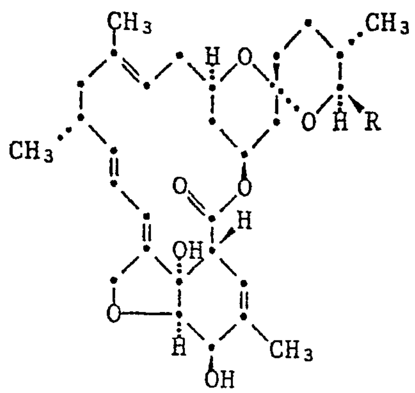

(II)

entweder direkt an der 5-OH-Gruppe mit einem reaktionsfähigen Säurederivat der Formel VI,

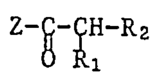

(VI),

zu Verbindungen der Formel I verestert, oder indem man eine Verbindung der Formel II zuerst durch Umsetzung mit einem reaktionsfähigen Säurederivat der Formel III,

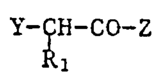

(III)

in eine Verbindung der Formel V

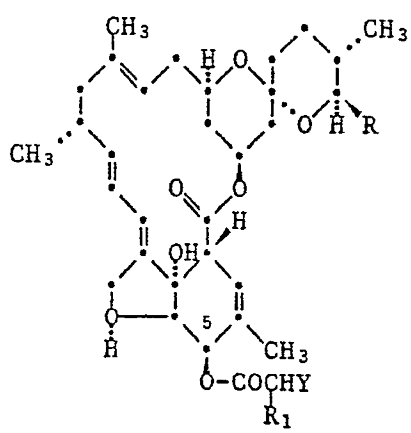

(V)

überführt und das Zwischenprodukt V in einer Folgereaktion mit einem Alkalisalz der (Thio)säure IV

$$MXC(O)R_2 \qquad (IV)$$

in das Endprodukt der Formel I überführt, wobei die Substituenten R, $R_1$, $R_2$ und X wie unter Formel I definiert sind, Z für Halogen steht, Y für Halogen steht und M ein Alkalimetallkation repräsentiert.

8. Mittel zur Bekämpfung von parasitären Schädlingen, dadurch gekennzeichnet, dass es neben üblichen Formulierungshilfsstoffen mindestens eine Verbindung der Formel I

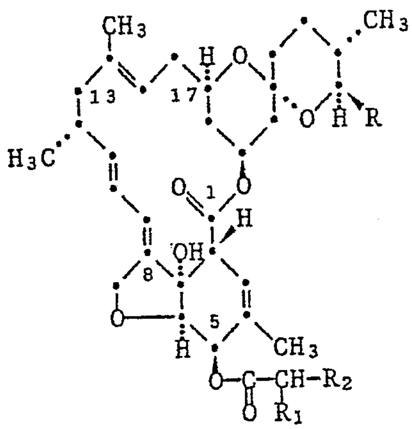

(I)

worin

R für Methyl, Ethyl, Isopropyl oder sek.-Butyl steht;

$R_1$ für Wasserstoff, Fluor oder $C_1$-$C_4$-Alkyl steht;

$R_2$ eine der Gruppen

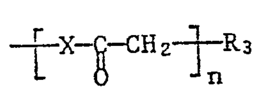 oder 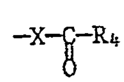 repräsentiert; wobei

n eine der Zahlen 2 bis 6 bedeutet;

X für Sauerstoff oder Schwefel steht ;

$R_3$ für Halogen steht; und

$R_4$ Wasserstoff, $C_1$-$C_{12}$-Alkoxy, $C_3$-$C_7$-Cycloalkoxy, $C_2$-$C_6$-Alkenyloxy, unsubstituiertes oder durch Halogen, $C_1$-$C_4$-Alkoxy, Hydroxy und/oder COOG substituiertes $C_1$-$C_{18}$-Alkyl, wobei G für Wasserstoff, ein Alkalimetall- oder Erdalkalimetallkation steht, unsubstituiertes oder durch Halogen und/oder $C_1$-$C_4$-Alkyl substituiertes $C_3$-$C_7$-Cycloalkyl, unsubstituiertes oder durch Halogen substituiertes $C_2$-$C_{18}$-Alkenyl, unsubstituiertes oder durch Halogen substituiertes $C_2$-$C_{18}$-Alkinyl, unsubstituiertes oder durch Halogen, $C_1$-$C_4$-Alkyl, $C_1$-$C_4$-Alkoxy, Nitro, Cyano und/oder $C_1$-$C_4$-Haloalkyl substituiertes Phenyl oder einen unsubstituierten oder durch Oxo und/oder ein- bis dreifach durch Halogen, $C_1$-$C_4$-Alkyl, $C_1$-$C_4$-Alkoxy und/oder $C_1$-$C_4$-Haloalkyl substituierten fünf- oder sechsgliedrigen, ungesättigten oder gesättigten heterocyclischen Ring mit ein- bis drei Heteroatomen ausgewählt aus der Reihe Stickstoff, Sauerstoff und Schwefel bedeutet, enthält.

9. Mittel gemäss Anspruch 8, dadurch gekennzeichnet, dass es als Verbindung der Formel I mindestens eine Verbindung gemäss den Ansprüchen 2 bis 6 enthält.

10. Verfahren zur Bekämpfung von Schädlingen an Nutztieren und Pflanzen, dadurch gekennzeichnet, dass man eine aktive Menge einer Verbindung der Formel I

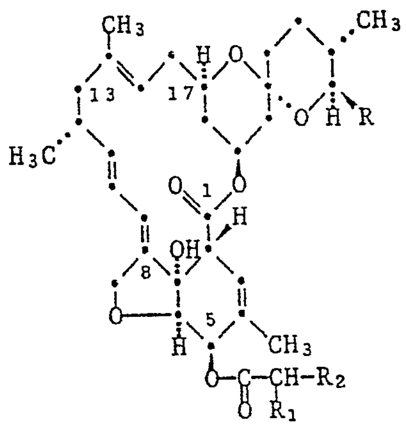

(I)

worin

R für Methyl, Ethyl, Isopropyl oder sek.-Butyl steht;

$R_1$ für Wasserstoff, Fluor oder $C_1$-$C_4$-Alkyl steht;

$R_2$ eine der Gruppen

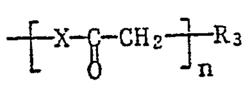 oder 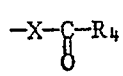 repräsentiert; wobei

n eine der Zahlen 2 bis 6 bedeutet;

X für Sauerstoff oder Schwefel steht ;

$R_3$ für Halogen steht; und

$R_4$ Wasserstoff, $C_1$-$C_{12}$-Alkoxy, $C_3$-$C_7$-Cycloalkoxy, $C_2$-$C_6$-Alkenyloxy, unsubstituiertes oder durch Halogen, $C_1$-$C_4$-Alkoxy, Hydroxy und/oder COOG substituiertes $C_1$-$C_{18}$-Alkyl, wobei G für Wasserstoff, ein Alkalimetall- oder Erdalkalimetallkation steht, unsubstituiertes oder durch Halogen und/oder $C_1$-$C_4$-Alkyl substituiertes $C_3$-$C_7$-Cycloalkyl, unsubstituiertes oder durch Halogen substituiertes $C_2$-$C_{18}$-Alkenyl, unsubstituiertes oder durch Halogen substituiertes $C_2$-$C_{18}$-Alkinyl, unsubstituiertes oder durch Halogen, $C_1$-$C_4$-Alkyl, $C_1$-$C_4$-Alkoxy, Nitro, Cyano und/oder $C_1$-$C_4$-Haloalkyl substituiertes Phenyl oder einen unsubstituierten oder durch Oxo und/oder ein- bis dreifach durch Halogen, $C_1$-$C_4$-Alkyl, $C_1$-$C_4$-Alkoxy und/oder $C_1$-$C_4$-Haloalkyl substituierten fünf- oder sechsgliedrigen, ungesättigten oder gesättigten heterocyclischen Ring mit ein- bis drei Heteroatomen ausgewählt aus der Reihe Stickstoff, Sauerstoff und Schwefel bedeutet, auf oder in das Tier, auf die Pflanze oder deren Lebensraum appliziert.

11. Verfahren gemäss Anspruch 10 zur Bekämpfung von Endoparasiten im Warmblüter.

12. Verfahren gemäss Anspruch 11,dadurch gekennzeichnet, dass es sich bei den Endoparasiten um Nematoden handelt.

FO 7.5/HL/cc*

Patentansprüche für den Vertragsstaat Oesterreich

1. Schädlingsbekämpfungsmittel enthaltend nebem üblichen Formulierungshilfsstoffen mindestens eine Verbindungen der Formel I

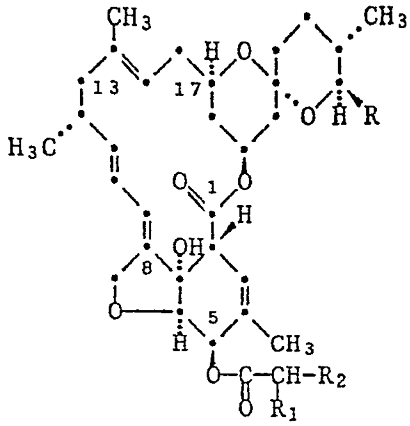

(I)

worin

R für Methyl, Ethyl, Isopropyl oder sek.-Butyl steht;

$R_1$ für Wasserstoff, Fluor oder $C_1$-$C_4$-Alkyl steht;

$R_2$ eine der Gruppen

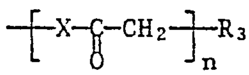 oder 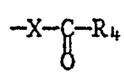 repräsentiert; wobei

n eine der Zahlen 2 bis 6 bedeutet;

X für Sauerstoff oder Schwefel steht ;

$R_3$ für Halogen steht; und

$R_4$ Wasserstoff, $C_1$-$C_{12}$-Alkoxy, $C_3$-$C_7$-Cycloalkoxy, $C_2$-$C_6$-Alkenyloxy, unsubstituiertes oder durch Halogen, $C_1$-$C_4$-Alkoxy, Hydroxy und/oder COOG substituiertes $C_1$-$C_{18}$-Alkyl, wobei G für Wasserstoff, ein Alkalimetall- oder Erdalkalimetallkation steht, unsubstituiertes oder durch Halogen und/oder $C_1$-$C_4$-Alkyl substituiertes $C_3$-$C_7$-Cycloalkyl, unsubstituiertes oder durch Halogen substituiertes $C_2$-$C_{18}$-Alkenyl, unsubstituiertes oder durch Halogen substituiertes $C_2$-$C_{18}$-Alkinyl, unsubstituiertes oder durch Halogen, $C_1$-$C_4$-Alkyl, $C_1$-$C_4$-Alkoxy, Nitro, Cyano und/oder $C_1$-$C_4$-Haloalkyl substituiertes Phenyl oder einen unsubstituierten oder durch Oxo und/oder ein- bis dreifach durch Halogen, $C_1$-$C_4$-Alkyl, $C_1$-$C_4$-Alkoxy und/oder $C_1$-$C_4$-Haloalkyl substituierten fünf-

oder sechsgliedrigen, ungesättigten oder gesättigten heterocyclischen Ring mit ein- bis drei Heteroatomen ausgewählt aus der Reihe Stickstoff, Sauerstoff und Schwefel bedeutet.

2. Mittel nach Anspruch 1, enthaltend mindestens eine Verbindung der Formel I, worin R für Methyl, Ethyl, Isopropyl oder sek.-Butyl steht;
$R_1$ für Wasserstoff, Fluor oder Methyl steht:
$R_2$ eine der Gruppen

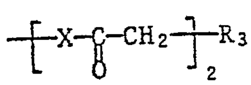 oder 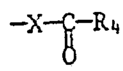 repräsentiert;

wobei X für Sauerstoff oder Schwefel steht;
$R_3$ für Halogen steht; und $R_4$ für Wasserstoff, $C_1$-$C_4$-Alkoxy, $C_3$-$C_6$-Cycloalkoxy, $C_2$-$C_6$-Alkenyloxy, unsubstituiertes oder durch Halogen, $C_1$-$C_4$-Alkoxy, Hydroxy und/oder COOG substituiertes $C_1$-$C_6$-Alkyl, wobei G für Wasserstoff oder ein Alkalimetallkation steht, $C_3$-$C_6$-Cycloalkyl, unsubstituiertes oder durch Halogen substituiertes $C_2$-$C_6$-Alkenyl, unsubstituiertes oder durch Halogen substituiertes $C_2$-$C_6$-Alkinyl oder unsubstituiertes oder durch Halogen, Methyl, Methoxy, Nitro, Cyano und/oder $CF_3$ substituiertes Phenyl steht.

3. Mittel nach Anspruch 2, enthaltend mindestens eine Verbindung der Formel I, worin R und $R_1$ wie in Anspruch 2 definiert sind; $R_2$ eine der Gruppen

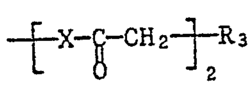 oder 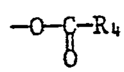 repräsentiert;

wobei $R_3$ für Fluor oder Chlor steht, $R_4$ für $C_1$-$C_4$-Alkoxy, $C_3$-$C_6$-Cyclolkoxy, $C_2$-$C_6$-Alkenyloxy, unsubstituiertes oder durch Fluor, Chlor, $C_1$-$C_4$-Alkoxy, Hydroxy oder COOH substituiertes $C_1$-$C_6$-Alkyl, $C_3$-$C_6$-Cycloalkyl, unsubstituiertes oder durch Fluor oder Chlor substituiertes $C_2$-$C_6$-Alkenyl, unsubstituiertes oder durch Fluor oder

Chlor substituiertes $C_2$-$C_6$-Alkinyl oder unsubstituiertes oder durch Fluor, Chlor, Methyl, Methoxy, Nitro, Cyano und/oder $CF_3$ substituiertes Phenyl steht.

4. Mittel nach Anspruch 1, enthaltend mindestens eine Verbindung der Formel I, worin R und $R_1$ wie in Anspruch 1 definiert sind; $R_2$ die Gruppe

$-X-\underset{\underset{O}{\|}}{C}-R_4$ repräsentiert, wobei

X für Sauerstoff oder Schwefel steht; $R_4$ einen unsubstituierten oder durch Oxo und/oder ein- bis dreifach durch Halogen, $C_1$-$C_4$-Alkyl, $C_1$-$C_4$-Alkoxy und/oder $C_1$-$C_4$-Haloalkyl substituierten fünf-oder sechsgliedrigen, ungesättigten oder gesättigten heterocyclischen Ring mit ein- bis drei Heteroatomen ausgewählt aus der Reihe Stickstoff, Sauerstoff und Schwefel bedeutet.

5. Mittel nach Anspruch 4, enthaltend mindestens eine Verbindung der Formel I, worin R und $R_1$ wie in Anspruch 1 definiert sind; $R_2$ die Gruppe

$-O-\underset{\underset{O}{\|}}{C}-R_4$ repräsentiert,

$R_4$ einen unsubstituierten oder durch Oxo und/oder ein- bis dreifach durch Halogen, $C_1$-$C_4$-Alkyl, $C_1$-$C_4$-Alkoxy und/oder $C_1$-$C_4$-Haloalkyl substituierten fünf-oder sechsgliedrigen, ungesättigten oder gesättigten heterocyclischen Ring mit ein- bis drei Heteroatomen ausgewählt aus der Reihe Stickstoff, Sauerstoff und Schwefel bedeutet, wobei der heterocyclische Ring ausgewählt ist aus der Gruppe von Furan, Thiophen, Pyrrol, Isoxazol, Isothiazol, Furazan, Imidazol, 1,2,4-Triazol, 1,2,3-Triazol, Pyrazol, Pyrrolin, Oxazol, Thiazol, Thiadiazole, Pyrazolin, Thiazolin, Pyrazolidin, Pyrrolidin, Oxazolidin, Thiazolidin, Oxadiazol, Imidazolin, Imidazolidin, Pyrazolidin, Tetrahydrofuran; und typische sechsgliedgrige heterocyclische Ringe sind Pyridin, Pyridazin, Pyrimidin, Pyrazin, Thiazin, Thiadiazine, Pyrane, Piperidin, Piperazin, Morpholin,

Perhydrothiazin, Dioxan sowie ihre teilhydrierten bzw. teilgesättigten Homologen, Bytrolactone, Valerolactone, Butyrolactam, Valerolactam, oder Camphan.

6. Mittel nach Anspruch 1, enthaltend mindestens eine Verbindung der Formel I, ausgewählt aus der Reihe:

5-O-Acetoxy-acetyl-milbemycin D
5-O-Acetoxy-acetyl-milbemycin $A_4$
5-O-Acetoxy-acetyl-milbemycin $A_3$
5-O-Acetoxy-acetyl-13-deoxy-22,23-dihydro-avermectin-Bla-aglycon
5-O-(3,4-Dihydro-2H-pyran-2-yl)carboxy-acetyl-milbemycin D
5-O-(3,4-Dihydro-2H-pyran-2-yl)carboxy-acetyl-milbemycin $A_4$
5-O-(3,4-Dihydro-2H-pyran-2-yl)carboxy-acetyl-milbemycin $A_3$
5-O-(5-Pyrrolidon-2-yl)carboxy-acetyl-milbemycin D
5-O-(5-Pyrrolidon-2-yl)carboxy-acetyl-milbemycin $A_4$
5-O-((S)-5-Pyrrolidon-2-yl)carboxy-acetyl-milbemycin $A_4$
5-O-((R)-5-Pyrrolidon-2-yl)carboxy-acetyl-milbemycin $A_4$
5-O-Camphanoyloxyacetyl-milbemycin $A_4$
5-O-((S)-2-Hydroxy-propionyloxy)acetyl-milbemycin D
5-O-Chloracetoxyacetyl-milbemycin D
5-O-Chloracetoxy-acetoxy-acetyl-milbemycin D
5-O-Chloracetoxy-acetoxy-acetoxy-acetyl-milbemycin D
5-O-Acetylthio-acetyl-milbemycin D.
5-O-Fluoracetoxy-acetyl-milbemycin $A_4$
5-O-Formyloxy-acetyl-milbemycin $A_4$
5-O-Benzoyloxy-acetyl-milbemycin $A_4$
5-O-Propionyloxy-acetyl-milbemycin $A_4$
5-O-Methoxyacetoxy-acetyl-milbemycin $A_4$
5-O-(Acetoxy)-fluoracetyl-milbemycin $A_4$
5-O-(2-Acetoxy)-propionyl-milbemycin $A_4$
5-O-(2-Acetoxy)-butanoyl-milbemycin $A_4$
5-O-Cyclopropyl-carbonyloxy-acetyl-milbemycin $A_4$
5-O-Methoxy-carbonyloxy-acetyl-milbemycin $A_4$
5-O-(3-Chlorbenzoyloxy)-acetyl-milbemycin D
5-O-(3-Chlorbenzoyloxy)-acetyl-milbemycin $A_4$

5-O-(3-Chlorbenzoyloxy)-acetyl-milbemycin $A_3$

und

5-O-((S)-2-Hydroxy-propionyloxy)acetyl-milbemycin $A_4$.

7. Verfahren zur Herstellung von Verbindungen der Formel I

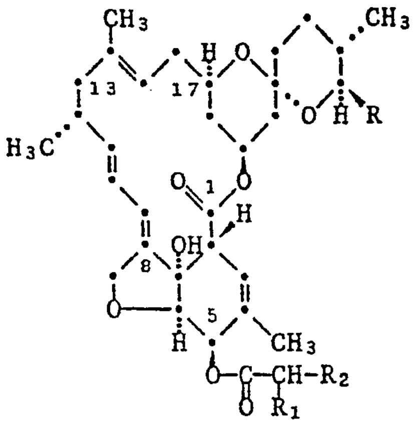

(I)

worin

R für Methyl, Ethyl, Isopropyl oder sek.-Butyl steht;

$R_1$ für Wasserstoff, Fluor oder $C_1$-$C_4$-Alkyl steht;

$R_2$ eine der Gruppen

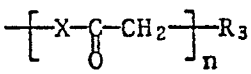 oder 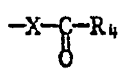 repräsentiert; wobei

n eine der Zahlen 2 bis 6 bedeutet;

X für Sauerstoff oder Schwefel steht ;

$R_3$ für Halogen steht; und

$R_4$ Wasserstoff, $C_1$-$C_{12}$-Alkoxy, $C_3$-$C_7$-Cycloalkoxy, $C_2$-$C_6$-Alkenyloxy, unsubstituiertes oder durch Halogen, $C_1$-$C_4$-Alkoxy, Hydroxy und/oder COOG substituiertes $C_1$-$C_{18}$-Alkyl, wobei G für Wasserstoff, ein Alkalimetall- oder Erdalkalimetallkation steht, unsubstituiertes oder durch Halogen und/oder $C_1$-$C_4$-Alkyl substituiertes $C_3$-$C_7$-Cycloalkyl, unsubstituiertes oder durch Halogen substituiertes $C_2$-$C_{18}$-Alkenyl, unsubstituiertes oder durch Halogen substituiertes $C_2$-$C_{18}$-Alkinyl, unsubstituiertes oder durch Halogen, $C_1$-$C_4$-Alkyl, $C_1$-$C_4$-Alkoxy, Nitro, Cyano und/oder $C_1$-$C_4$-Haloalkyl substituiertes Phenyl oder einen unsubstituierten oder durch Oxo und/oder ein- bis dreifach durch Halogen, $C_1$-$C_4$-

Alkyl, $C_1$-$C_4$-Alkoxy und/oder $C_1$-$C_4$-Haloalkyl substituierten fünf- oder sechsgliedrigen, ungesättigten oder gesättigten heterocyclischen Ring mit ein- bis drei Heteroatomen ausgewählt aus der Reihe Stickstoff, Sauerstoff und Schwefel bedeutet, dadurch gekennzeichnet, dass man eine Verbindung der Formel II

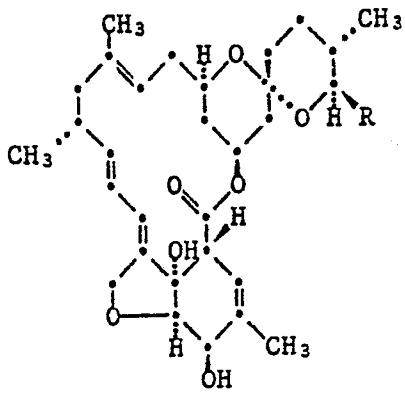

(II)

entweder direkt an der 5-OH-Gruppe mit einem reaktionsfähigen Säurederivat der Formel VI,

$$\underset{\underset{O}{\|}}{Z-C}-\underset{\underset{R_1}{|}}{CH}-R_2 \qquad \text{(VI)},$$

zu Verbindungen der Formel I verestert, oder indem man eine Verbindung der Formel II zuerst durch Umsetzung mit einem reaktionsfähigen Säurederivat der Formel III,

$$Y-\underset{\underset{R_1}{|}}{CH}-CO-Z \qquad \text{(III)}$$

in eine Verbindung der Formel V

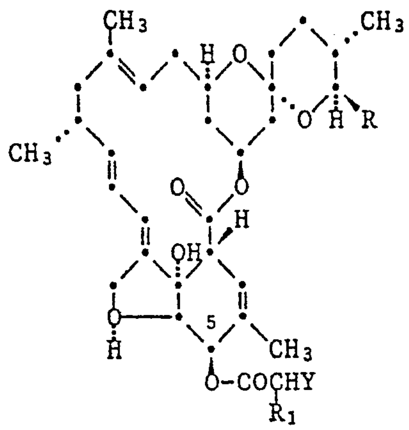

(V)

überführt und das Zwischenprodukt V in einer Folgereaktion mit einem Alkalisalz der (Thio)säure IV

$$MXC(O)R_2 \qquad (IV)$$

in das Endprodukt der Formel I überführt, wobei die Substituenten R, $R_1$, $R_2$ und X wie unter Formel I definiert sind, Z für Halogen steht, Y für Halogen steht und M ein Alkalimetallkation repräsentiert.

8. Verfahren zur Bekämpfung von Schädlingen an Nutztieren und Pflanzen, dadurch gekennzeichnet, dass man eine aktive Menge einer Verbindung der Formel I

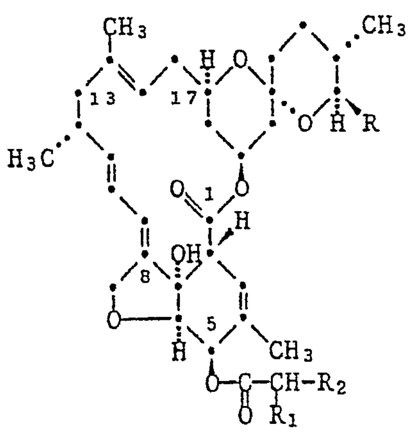

(I)

worin

R für Methyl, Ethyl, Isopropyl oder sek.-Butyl steht;

$R_1$ für Wasserstoff, Fluor oder $C_1$-$C_4$-Alkyl steht;

$R_2$ eine der Gruppen

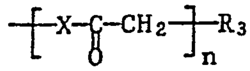 oder 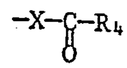 repräsentiert; wobei

n eine der Zahlen 2 bis 6 bedeutet;

X für Sauerstoff oder Schwefel steht ;

$R_3$ für Halogen steht; und

$R_4$ Wasserstoff, $C_1$-$C_{12}$-Alkoxy, $C_3$-$C_7$-Cycloalkoxy, $C_2$-$C_6$-Alkenyloxy, unsubstituiertes oder durch Halogen, $C_1$-$C_4$-Alkoxy, Hydroxy und/oder COOG substituiertes $C_1$-$C_{18}$-Alkyl, wobei G für Wasserstoff, ein Alkalimetall- oder Erdalkalimetallkation steht, unsubstituiertes oder durch Halogen und/oder $C_1$-$C_4$-Alkyl substituiertes $C_3$-$C_7$-Cycloalkyl, unsubstituiertes oder durch Halogen substituiertes $C_2$-$C_{18}$-Alkenyl, unsubstituiertes oder durch Halogen substituiertes $C_2$-$C_{18}$-Alkinyl, unsubstituiertes oder durch Halogen, $C_1$-$C_4$-Alkyl, $C_1$-$C_4$-Alkoxy, Nitro, Cyano und/oder $C_1$-$C_4$-Haloalkyl substituiertes Phenyl oder einen unsubstituierten oder durch Oxo und/oder ein- bis dreifach durch Halogen, $C_1$-$C_4$-Alkyl, $C_1$-$C_4$-Alkoxy und/oder $C_1$-$C_4$-Haloalkyl substituierten fünf- oder sechsgliedrigen, ungesättigten oder gesättigten heterocyclischen Ring mit ein- bis drei Heteroatomen ausgewählt aus der Reihe Stickstoff, Sauerstoff und Schwefel bedeutet, auf oder in das Tier, auf die Pflanze oder deren Lebensraum appliziert.

9. Verfahren gemäss Anspruch 8 zur Bekämpfung von Endoparasiten im Warmblüter.

10. Verfahren gemäss Anspruch 9,dadurch gekennzeichnet, dass es sich bei den Endoparasiten um Nematoden handelt.